# EUROPEAN PATENT APPLICATION

(11) **EP 4 410 789 A1**
(43) Date of publication of application: **07.08.2024**
(21) Application number: 22875707.6
(22) Date of filing: 31.08.2022
(51) Int. Cl.: C07D 405/10, C09K 11/06, H10K 50/00, H10K 50/15

(54) **COMPOUND, COMPOSITION, HOST MATERIAL, ELECTRON BARRIER MATERIAL AND ORGANIC LIGHT EMITTING ELEMENT**

(30) Priority: 28.09.2021 JP 2021157765; 30.05.2022 JP 2022087873
(71) Applicant: Kyulux, Inc., Fukuoka-shi, Fukuoka 819-0388 (JP)
(72) Inventor: OZAWA Hiroaki, Fukuoka-shi, Fukuoka 819-0388 (JP); MORIO Momoko, Fukuoka-shi, Fukuoka 819-0388 (JP); GOTO Aiko, Fukuoka-shi, Fukuoka 819-0388 (JP); KASHIWAZAKI Takahiro, Fukuoka-shi, Fukuoka 819-0388 (JP); MORIMOTO Kei, Fukuoka-shi, Fukuoka 819-0388 (JP); HWANG Songhye, Fukuoka-shi, Fukuoka 819-0388 (JP)
(74) Representative: Zimmermann & Partner Patentanwälte mbB
(86) International application number: PCT/JP2022/032781
(87) International publication number: WO 2023/053835

(57) **Abstract**

A compound of the following formula is useful as a host material or an electron barrier material. R¹ to R⁵ are a deuterium atom or an alkyl group, but one or two among R⁴ and R⁵ are a phenyl group that may be substituted with an alkyl group. n1 and n3 to n5 are 0 to 4, and n2 is 0 to 3.

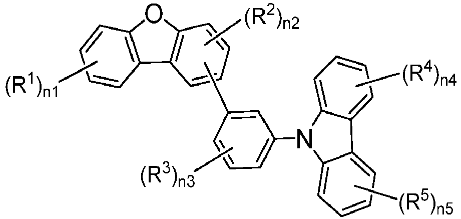

## Description

### Background Art

The present invention relates to a compound useful as a host material or an electron barrier material, a composition using the compound, and an organic light-emitting device.

Researches have been actively conducted to improve the luminous efficiency of light-emitting devices such as organic electroluminescence device (organic EL devices). In particular, various studies have been conducted to improve the luminous efficiency by newly developing and combining an electron transport material, a hole transport material, a light-emitting material, a host material, and the like configuring the organic electroluminescence device. Among them, an organic electroluminescence device using a delayed fluorescence material has been developed and has attracted attention (refer to Non-Patent Document 1)

The delayed fluorescence material is a material that causes inverse intersystem crossing to the excited singlet state from the excited triplet state in the excited state, and then, emits fluorescence when returning to the ground state from the excited singlet state. The fluorescence through such a route is observed later than the fluorescence (general fluorescence) from the excited singlet state that directly occurs from the ground state, and thus, is referred to as delayed fluorescence. Here, for example, when a light-emitting compound is excited by the injection of carriers, the occurrence probability of the excited singlet state and the excited triplet state is statistically 25%:75%, and thus, there is a limit to the improvement of the luminous efficiency only by the fluorescence from the excited singlet state that directly occurs. On the other hand, in the delayed fluorescence material, not only the excited singlet state but also the excited triplet state can be used in the fluorescence emission through the route via the inverse intersystem crossing, and thus, high luminous efficiency can be obtained compared to the general fluorescence material. The delayed fluorescence material having such features is generally used in a light-emitting layer of the organic electroluminescence device together with a host material, and actually contributes to the improvement of the luminous efficiency.

Non-Patent Document 1 Uoyama et al, Nature, 492, 234-238 (2012)

As the host material to be combined with the delayed fluorescence material, a compound having lowest excited singlet energy larger than that of the delayed fluorescence material is selected. However, even in a case where the host material that has been used in combination with a fluorescence material of the related art, which does not emit delayed fluorescence, is directly combined with the delayed fluorescence material, sufficient luminescent performance is not capable of being realized. In particular, in the organic electroluminescence device using the delayed fluorescence material, there is room for improvement in a driving voltage or emission lifetime. Thus, in the organic light-emitting device using the delayed fluorescence material, the present inventors have conducted examination in order to suppress the driving voltage and to extend the emission lifetime while achieving high luminous efficiency.

### Summary of Invention

The present inventors have conducted the intensive studies, and as a result, have found that when a material having a specific structure is used in combination with the delayed fluorescence material, it is possible to achieve lifetime extension while suppressing the driving voltage, and to realize high luminous efficiency. The invention is suggested on the basis of such findings, and has the following configurations.
[1] A compound represented by the following formula (1). 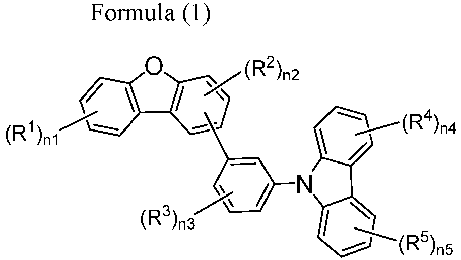 In the formula, each of R¹ to R³ independently represents a deuterium atom, or an alkyl group that may be deuterated, each of R⁴ and R⁵ independently represents a deuterium atom, an alkyl group that may be deuterated, or a phenyl group that may be substituted with an alkyl group that may be deuterated or a deuterium atom. Here, one or two among R⁴ and R⁵ are a phenyl group that may be substituted with an alkyl group that may be deuterated or a deuterium atom. R¹ to R⁵ are not bonded to other R¹ to R⁵ to form a ring structure, and adjacent R¹'s, adjacent R²'s, and adjacent R³'s are not bonded to each other to form a ring structure. Adjacent R⁴'s may be bonded to each other to form a benzofuro skeleton or a benzothieno skeleton, and adjacent R⁵'s may be bonded to each other to form a benzofuro skeleton or a benzothieno skeleton. Each of n1, n3, n4, and n5 independently represents any integer of 0 to 4, n2 represents any integer of 0 to 3, and a sum of n4 and n5 is 1 to 8.
[2] The compound described in [1], in which adjacent R⁴'s and adjacent R⁵'s are not bonded to each other to form a ring structure.
[3] The compound described in [1], in which adjacent R⁵'s are bonded to each other to form a benzofuro skeleton or a benzothieno skeleton.
[4] The compound described in any one of [1] to [3], in which each of R⁴ and R⁵ is independently a deuterium atom or a phenyl group, and the phenyl group may be substituted with a deuterium atom, or an alkyl group that may be deuterated.
[5] The compound described in any one of [1] to [4], in which each of only one R⁴ and one R⁵ is independently a phenyl group that may be substituted with an alkyl group that may be deuterated or a deuterium atom.
[6] The compound described in any one of [1] to [4], in which only one R⁴ is a phenyl group that may be substituted with an alkyl group that may be deuterated or a deuterium atom.
[7] The compound described in any one of [1] to [6], in which R¹ to R³ are a deuterium atom.
[8] The compound described in any one of [1] to [7], in which at least one of R⁴'s is a deuterium atom, and at least one of R⁵'s is a deuterium atom.
[9] The compound described in any one of [1] to [8], in which n1 to n3 are 0.
[10] The compound described in any one of [1] to [9], in which dibenzofuran in the formula (1) is bonded to a phenylene group in the formula (1) at a 2-position.
[11] The compound described in any one of [1] to [10], in which the compound is represented by the following formula (2). 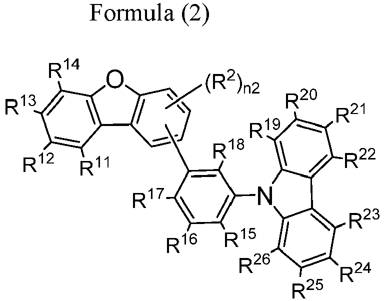 In the formula (2), R² represents an alkyl group that may be deuterated, and n2 represents an integer of 0 to 3. Each of R¹¹ to R¹⁸ independently represents a hydrogen atom, a deuterium atom, or an alkyl group that may be deuterated. Each of R¹⁹ to R²⁶ independently represents a hydrogen atom, a deuterium atom, an alkyl group that may be deuterated, or a phenyl group that may be substituted with an alkyl group that may be deuterated or a deuterium atom. Here, one or two among R¹⁹ to R²⁶ are a phenyl group that may be substituted with an alkyl group that may be deuterated or a deuterium atom. R¹⁹ and R²⁰, R²⁰ and R²¹, R²¹ and R²², R²³ and R²⁴, R²⁴ and R²⁵, and R²⁵ and R²⁶ may be bonded to each other to form a benzofuro skeleton or a benzothieno skeleton.
[12] The compound described in [11], in which none of adjacent R²'s, R¹¹ and R¹², R¹² and R¹³, R¹³ and R¹⁴, R¹⁵ and R¹⁶, R¹⁶ and R¹⁷, R¹⁹ and R²⁰, R²⁰ and R²¹, R²¹ and R²², R²³ and R²⁴, R²⁴ and R²⁵, and R²⁵ and R²⁶ are bonded to each other to form a ring structure.
[13] The compound described in [11] or [12], in which at least one of R²¹ and R²⁴ is a phenyl group that may be substituted with an alkyl group that may be deuterated or a deuterium atom.
[14] A host material including the compound described in any one of [1] to [13].
[15] The host material described in [14] for use together with a delayed fluorescence material.
[16] An electron barrier material including the compound described in any one of [1] to [13], in particular, an electron barrier material for use in an electron barrier layer in contact with a light-emitting layer containing a delayed fluorescence material.
[17] A composition formed by doping the compound described in any one of [1] to [13] with a delayed fluorescence material.
[18] The composition described in [17], which is in the shape of a film.
[19] The composition described in [17] or [18], in which the delayed fluorescence material is a compound having a cyanobenzene structure in which the number of cyano groups substituted with the benzene ring is 1.
[20] The composition described in [17] or [18], in which the delayed fluorescence material is a compound having a dicyanobenzene structure in which the number of cyano groups substituted with the benzene ring is 2.
[21] The composition described in any one of [17] to [20], further including a fluorescent compound having lowest excited singlet energy lower than that of the host material and the delayed fluorescence material.
[22] An organic light-emitting device including a layer containing the composition described in any one of [17] to [21].
[23] The organic light-emitting device described in [22], in which the layer consists of atoms selected from the group consisting of a carbon atom, a hydrogen atom, a nitrogen atom, an oxygen atom, a sulfur atom, a boron atom, and a halogen atom.
[24] The organic light-emitting device described in [23], in which the layer consists of atoms selected from the group consisting of a carbon atom, a hydrogen atom, a nitrogen atom, an oxygen atom, and a sulfur atom.
[25] The organic light-emitting device described in any one of [22] to [24], which is an organic electroluminescence device.
[26] The organic light-emitting device described in any one of [22] to [25], in which the composition does not contain the fluorescent compound, and the largest component of light emitted from the device is light emitted from the delayed fluorescence material.
[27] The organic light-emitting device described in any one of [22] to [25], in which the composition contains the fluorescent compound, and the largest component of light emitted from the device is light emitted from the fluorescent compound.

In the case of using the compound of the invention, it is possible to provide the organic light-emitting device that is driven at a low voltage and has a long lifetime.

### Brief Description of Drawings

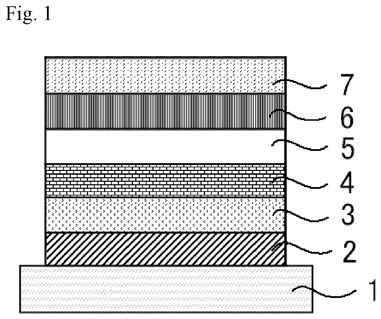

FIG. 1 is a schematic sectional view illustrating a configuration example of a layer of an organic electroluminescence device.

### Description of Embodiments

Hereinafter, the contents of the invention will be described in detail. The descriptions on constituent elements to be described below may be made on the basis of representative embodiments or specific examples of the invention, but the invention is not limited to such embodiments or specific examples. The numerical value range represented by using "to" in the present specification means a range including numerical values described before and after "to", as the lower limit value and the upper limit value. Further, the isotope species of hydrogen atoms present in the molecule of a compound used in the invention are not particularly limited.

### (Compound represented by formula (1))

In the invention, a compound represented by the following formula (1) is used.

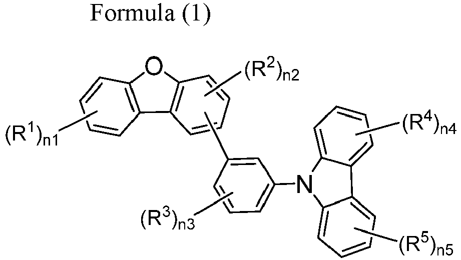

In the formula (1), each of R¹ to R³ independently represents a deuterium atom, or an alkyl group that may be deuterated, and each of R⁴ and R⁵ independently represents a deuterium atom, an alkyl group that may be deuterated, or a phenyl group that may be substituted with an alkyl group that may be deuterated or a deuterium atom. Each of n1, n3, n4, and n5 independently represents any integer of 0 to 4, n2 represents any integer of 0 to 3, and a sum of n4 and n5 is 1 to 8.

The "alkyl group" in this application may take any of linear, branched, and cyclic shapes. Further, two or more types of the linear portion, the cyclic portion, and the branched portion may be mixed. The number of carbon atoms of the alkyl group may be, for example, 1 or more, 2 or more, or 4 or more. Further, the number of carbon atoms may be 30 or less, 20 or less, 10 or less, 6 or less, or 4 or less. Specific examples of the alkyl group include a methyl group, an ethyl group, a n-propyl group, an isopropyl group, a n-butyl group, an isobutyl group, a tert-butyl group, a n-pentyl group, an isopentyl group, a n-hexyl group, a cyclopentyl group, a cyclohexyl group, and a cycloheptyl group. In one aspect of the invention, the number of carbon atoms of the alkyl group is 1 to 4. In one aspect of the invention, the alkyl group is a methyl group. In one aspect of the invention, the alkyl group is an isopropyl group. In one aspect of the invention, the alkyl group is a tert-butyl group. When there are a plurality of alkyl groups in the molecule represented by the formula (1), such alkyl groups may be the same as or different from each other. In one aspect of the invention, all the alkyl groups in the molecule represented by the formula (1) are the same. The number of alkyl groups in the molecule represented by the formula (1) can be 0 or more, 1 or more, 2 or more, 4 or more, or 8 or more. The number of alkyl groups in the molecule represented by the formula (1) may be 20 or less, 10 or less, 5 or less, or 3 or less. The number of alkyl groups in the molecule represented by the formula (1) may be 0. The number of alkyl groups mentioned herein also includes the number of alkyl groups substituted with a phenyl group.

The "phenyl group that may be substituted with an alkyl group" that may be possessed by R⁴ and R⁵ means that at least one of five hydrogen atoms present in the phenyl group may be substituted with an alkyl group. In one aspect of the invention, the phenyl group is substituted with 0 to 3 alkyl groups. For example, the phenyl group may be substituted with 0 to 2 alkyl groups. In one aspect of the invention, the phenyl group is not substituted with an alkyl group. The number of carbon atoms of the alkyl group substituted with the phenyl group is preferably 1 to 6, and more preferably 1 to 4. Examples thereof include a 4-methyl phenyl group, a 3-methyl phenyl group, a 3,5-dimethyl phenyl group, a 2,4,6-trimethyl phenyl group, a 2,3,4,5,6-heptamethyl phenyl group, a 4-isopropyl phenyl group, a 3-isopropyl phenyl group, a 3,5-diisopropyl phenyl group, a 4-tert-butyl phenyl group, a 3-tert-butyl phenyl group, and a 3,5-ditert-butyl phenyl group. Further, a group in which all of hydrogen atoms present in an alkyl substituent in such specific examples are substituted with a deuterium atom can also be exemplified. Further, a group in which all of hydrogen atoms present in such specific examples are substituted with a deuterium atom can also be exemplified.

The "alkyl group that may be deuterated" in this application means that at least one of the hydrogen atoms of the alkyl group may be substituted with a deuterium atom. All the hydrogen atoms of the alkyl group may be substituted with a deuterium atom. For example, a methyl group that may be deuterated includes CH₃, CDH₂, CD₂H, and CD₃. The "alkyl group that may be deuterated" is preferably an alkyl group that is not deuterated at all, or an alkyl group in which all the hydrogen atoms are substituted with a deuterium atom. In one aspect of the invention, as the "alkyl group that may be deuterated", an alkyl group that is not deuterated at all is selected. In one aspect of the invention, as the "alkyl group that may be deuterated", an alkyl group in which all the hydrogen atoms are substituted with a deuterium atom is selected.

A sum of n1 and n2 in the formula (1) is 0 to 7, and for example, may be in a range of 1 to 7. For example, the sum may be in a range of 1 to 4. In one aspect of the invention, n1 is an integer of 0 to 2, and n2 is an integer of 0 to 2. In one aspect of the invention, n1 is 0 or 1, and n2 is 0 or 1. In one aspect of the invention, both of n1 and n2 are 0. In one aspect of the invention, each of R¹ and R² independently represents a deuterium atom, or an alkyl group having 1 to 6 carbon atoms that may be deuterated, and preferably, each of R¹ and R² is independently a deuterium atom, or an alkyl group having 1 to 4 carbon atoms that may be deuterated. In one aspect of the invention, each of R¹ and R² is independently a deuterium atom, or a deuterated alkyl group. In one aspect of the invention, R¹ and R² are a deuterium atom. In one aspect of the invention, the sum of n1 and n2 is 7, and R¹ and R² are a deuterium atom.

Specific examples of a dibenzofuranyl group substituted with (R¹)ₙ₁ and (R²)ₙ₂ in the formula (1) are given below. Here, a structure that can be adopted in the invention is not construed as limiting to such specific examples. In this application, * represents a bond position to a meta-phenylene group. For description on a methyl group, CH₃ is omitted without indicating as CH₃. Thus, each of B2, B4, B6, and B8 has a methyl group.

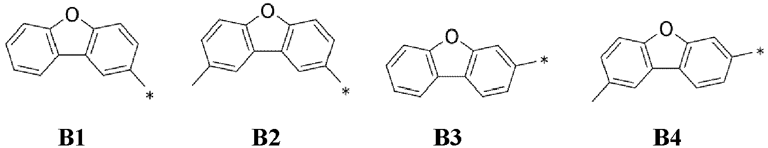

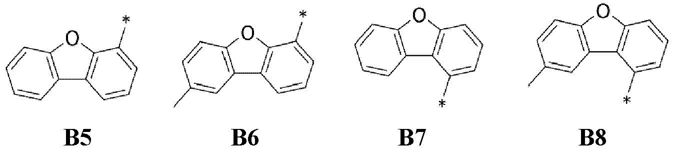

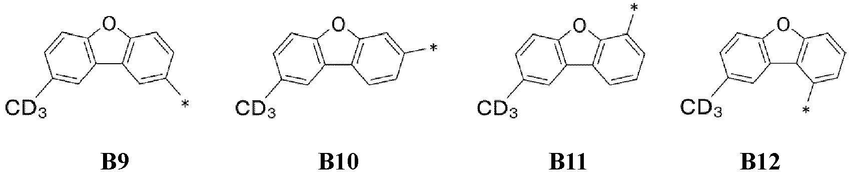

B1 to B8 in which all of hydrogen atoms are substituted with a deuterium atom are exemplified herein as B1(D) to B8(D), respectively.

n3 in the formula (1) is 0 to 4. In one aspect of the invention, n3 is 0. In one aspect of the invention, n3 is 1 to 4. In one aspect of the invention, R³ is a deuterium atom, or an alkyl group having 1 to 6 carbon atoms that may be deuterated, and preferably, each of R³'s is independently a deuterium atom, or an alkyl group having 1 to 4 carbon atoms that may be deuterated. In one aspect of the invention, R³ is a deuterium atom, or a deuterated alkyl group. In one aspect of the invention, R³ is a deuterium atom. In one aspect of the invention, n3 is 4, and R³ is a deuterium atom.

Specific examples of a meta-phenylene group substituted with (R³)ₙ₃ in the formula (1) are given below, but a structure that can be adopted in the invention is not construed as limiting to such specific examples. In the following specific examples, one of * represents a position bonded to carbazole in the formula (1), and the other represents a position bonded to dibenzofuran. In this application, description on a methyl group is omitted without indicating as CH₃. For example, each of Ar2 to Ar6 is substituted with a methyl group, Ar7 is substituted with an ethyl group, and Ar8 is substituted with an isopropyl group. t-Bu represents a tert-butyl group, and D represents a deuterium atom.

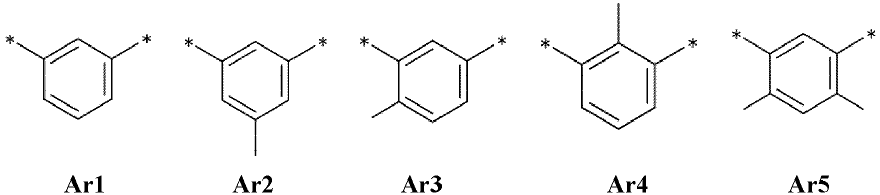

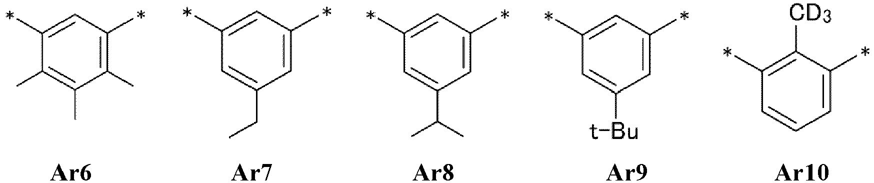

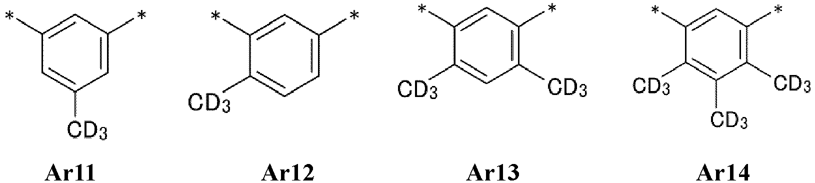

Ar1 to Ar9 in which all of hydrogen atoms are substituted with a deuterium atom are exemplified herein as Ar1(D) to Ar9(D), respectively.

One or two among R⁴ and R⁵ in the formula (1) are a phenyl group that may be substituted with an alkyl group that may be deuterated or a deuterium atom. In one aspect of the invention, the phenyl group mentioned herein is substituted only with an alkyl group that may be deuterated. In one aspect of the invention, the phenyl group mentioned herein is a phenyl group in which at least one hydrogen atom is substituted with a deuterium atom. In one aspect of the invention, the phenyl group mentioned herein is a phenyl group that is substituted not only an alkyl group that may be deuterated but also a deuterium atom. In one aspect of the invention, the phenyl group mentioned herein is an unsubstituted phenyl group. In one aspect of the invention, only one of R⁴'s is a phenyl group that may be substituted with an alkyl group that may be deuterated or a deuterium atom. In one aspect of the invention, only one of R⁵'s is a phenyl group that may be substituted with an alkyl group that may be deuterated or a deuterium atom. In one aspect of the invention, each of one R⁴ and one R⁵ is independently a phenyl group that may be substituted with an alkyl group that may be deuterated or a deuterium atom. In one aspect of the invention, at least one of R⁴'s is a deuterium atom, and at least one of R⁵'s is a deuterium atom.

Each of n4 and n5 in the formula (1) is 0 to 4, for example 1 to 4, for example 1 to 3, and for example 1 or 2, and the sum of n4 and n5 is 1 to 8, for example, may be 1 to 4. In one aspect of the invention, the sum of n4 and n5 is 1 or 2. In one aspect of the invention, the sum of n4 and n5 is 3 or 4.

In a preferred aspect of the invention, each of R¹ to R³ independently represents a deuterium atom, each of R⁴ and R⁵ independently represents a deuterium atom, or a phenyl group that may be substituted with a deuterium atom, and here, one or two among R⁴ and R⁵ are a phenyl group that may be substituted with a deuterium atom.

R¹ to R⁵ in the formula (1) are not bonded to other R¹ to R⁵ to form a ring structure. That is, R¹ is not bonded to R² to R⁵ to form a ring structure, and the same applies to R², R³, R⁴, and R⁵. Further, adjacent R¹'s are not bonded to each other to form a ring structure, adjacent R²'s are not bonded to each other to form a ring structure, and adjacent R³'s are not bonded to each other to form a ring structure. On the other hand, adjacent R⁴'s may be bonded to each other to form only a benzofuro skeleton or a benzothieno skeleton, and adjacent R⁵'s may be bonded to each other to form only a benzofuro skeleton or a benzothieno skeleton. When adjacent R⁴'s are bonded to each other to form a benzofuro skeleton or a benzothieno skeleton, and R⁴'s are not bonded to each other to form a benzofuro skeleton or a benzothieno skeleton, the compound of the formula (1) has a structure represented by the following formula (1a). Further, when adjacent R⁴'s are bonded to each other to form a benzofuro skeleton or a benzothieno skeleton, and adjacent R⁵'s are bonded to each other to form a benzofuro skeleton or a benzothieno skeleton, the compound of the formula (1) has a structure represented by the following formula (1b). In the formula (1a) and the formula (1b), each of X, X¹, and X² independently represents an oxygen atom or a sulfur atom. Each of R^{4a} and R^{5a} independently represents a deuterium atom, an alkyl group that may be deuterated, or a phenyl group that may be substituted with an alkyl group that may be deuterated or a deuterium atom. Each of n4a and n5a is independently any integer of 0 to 2, but a sum of n4a and n5 in the formula (1a) is 1 or more, and a sum of n4a and n5a in the formula (1b) is 1 or more. Further, one or two among R^{4a} and R⁵ in the formula (1a) and one or two among R^{4a} and R^{5a} in the formula (1b) are a phenyl group that may be substituted with an alkyl group that may be deuterated or a deuterium atom. Each of R⁶ and R⁷ independently represents a deuterium atom or a substituent (the substituent mentioned herein means an atom or a group of atoms other than a hydrogen atom and a deuterium atom). Each of preferable R⁶ and R⁷ is independently a deuterium atom, an alkyl group that may be deuterated, or a phenyl group that may be substituted with an alkyl group that may be deuterated or a deuterium atom. Adjacent R⁶'s and adjacent R⁷'s are not bonded to each other to form a ring structure. Each of n6 and n7 independently represents an integer of 0 to 4. For the description on R¹ to R³, R⁵, n1 to n3, and n5, corresponding description on the formula (1) can be referred to.

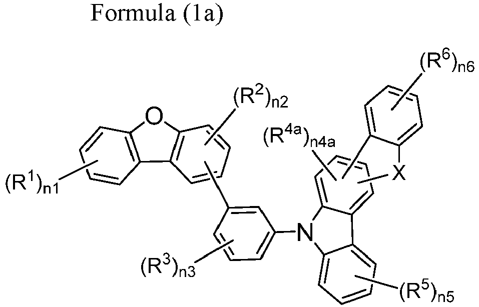

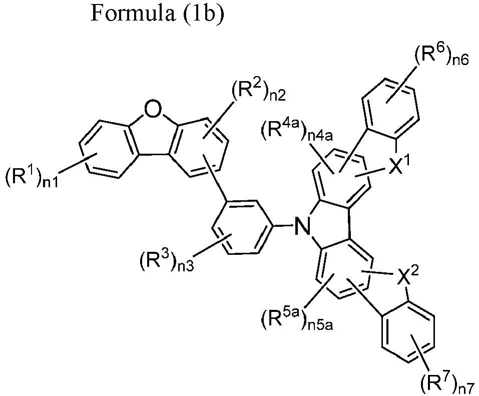

In the formula (1a), a single bond extends in a lower left direction from an ortho-position of a benzene ring bonded to the upper side of X. This single bond may be bonded to any of 1 to 4 positions of a carbazole structure. Further, X may also be bonded to any of 1 to 4 positions of the carbazole structure. Here, the single bond and X are bonded to adjacent carbon atoms constituting the skeleton of the carbazole structure, respectively. Thus, when the single bond is bonded to the 2-position, X is bonded to the 1-position or the 3-position. When the single bond is bonded to the 3-position, X is bonded to the 2-position or the 4-position. When the single bond is bonded to the 1-position, X is bonded to the 2-position. When the single bond is bonded to the 4-position, X is bonded to the 3-position. In the formula (1a), X is described to the right of the single bond, but it is assumed that the formula (1a) also includes a structure in which the single bond is positioned to the left of X. X¹ and X² in the formula (1b) are the same as X in the formula (1a).

As a benzofurocarbazole-9-yl group in the formula (1a), a substituted or unsubstituted benzofuro[2,3-a]carbazole-9-yl group can be adopted. Further, a substituted or unsubstituted benzofuro[3,2-a]carbazole-9-yl group can also be adopted. Further, a substituted or unsubstituted benzofuro[2,3-b]carbazole-9-yl group can also be adopted. Further, a substituted or unsubstituted benzofuro[3,2-b]carbazole-9-yl group can also be adopted. Further, a substituted or unsubstituted benzofuro[2,3-c]carbazole-9-yl group can also be adopted. Further, a substituted or unsubstituted benzofuro[3,2-c]carbazole-9-yl group can also be adopted.

As a benzothienocarbazole-9-yl group bonded to Ar in the formula (1a), a substituted or unsubstituted benzothieno[2,3-a]carbazole-9-yl group can be adopted. Further, a substituted or unsubstituted benzothieno[3,2-a]carbazole-9-yl group can also be adopted. Further, a substituted or unsubstituted benzothieno[2,3-b]carbazole-9-yl group can also be adopted. Further, a substituted or unsubstituted benzothieno[3,2-b]carbazole-9-yl group can also be adopted. Further, a substituted or unsubstituted benzothieno[2,3-c]carbazole-9-yl group can also be adopted. Further, a substituted or unsubstituted benzothieno[3,2-c]carbazole-9-yl group can also be adopted.

As a bisbenzofurocarbazole-9-yl group in the formula (1b), a substituted or unsubstituted bisbenzofuro[2,3-a:2',3'-f]carbazole-9-yl group can be adopted. Further, a substituted or unsubstituted bisbenzofuro[3,2-a:3',2'-f]carbazole-9-yl group can also be adopted. Further, a substituted or unsubstituted bisbenzofuro[2,3-b:2',3'-e]carbazole-9-yl group can also be adopted. Further, a substituted or unsubstituted bisbenzofuro[3,2-b:3',2'-e]carbazole-9-yl group can also be adopted. Further, a substituted or unsubstituted bisbenzofuro[2,3-c:2',3'-d]carbazole-9-yl group can also be adopted. Further, a substituted or unsubstituted bisbenzofuro[3,2-c:3',2'-d]carbazole-9-yl group can also be adopted.

As a bisbenzothienocarbazole-9-yl group in the formula (1b), a substituted or unsubstituted bisbenzothieno[2,3-a:2',3'-f]carbazole-9-yl group can be adopted. Further, a substituted or unsubstituted bisbenzothieno[3,2-a:3',2'-f]carbazole-9-yl group can also be adopted. Further, a substituted or unsubstituted bisbenzothieno[2,3-b:2',3'-e]carbazole-9-yl group can also be adopted. Further, a substituted or unsubstituted bisbenzothieno[3,2-b:3',2'-e]carbazole-9-yl group can also be adopted. Further, a substituted or unsubstituted bisbenzothieno[2,3-c:2',3'-d]carbazole-9-yl group can also be adopted. Further, a substituted or unsubstituted bisbenzothieno[3,2-c:3',2'-d]carbazole-9-yl group can also be adopted.

In one aspect of the invention, n4a in the formula (1a) is 0. In one aspect of the invention, n4a is 0, n5 is 1 or 2, and at least one of two R⁵'s is a phenyl group that may be substituted with an alkyl group that may be deuterated or a deuterium atom. In one aspect of the invention, n6 is 0, or n6 is 4, and four R⁶'s are a deuterium atom. In one aspect of the invention, n6 is 1 or 2, and at least one R⁶ is a phenyl group that may be substituted with an alkyl group that may be deuterated or a deuterium atom. In one aspect of the invention, X is an oxygen atom. In one aspect of the invention, X is a sulfur atom.

In one aspect of the invention, the sum of n4a and n5a in the formula (1b) is 1 or 2. In one aspect of the invention, only one among R^{4a} and R^{5a} is a phenyl group that may be substituted with an alkyl group that may be deuterated or a deuterium atom. In one aspect of the invention, n6 is 0, or n6 is 4, and four R⁶'s are a deuterium atom. In one aspect of the invention, n7 is 0, or n7 is 4, and four R⁷'s are a deuterium atom. In one aspect of the invention, n6 is 1 or 2, and at least one R⁶ is a phenyl group that may be substituted with an alkyl group that may be deuterated or a deuterium atom. In one aspect of the invention, n7 is 1 or 2, and at least one R⁷ is a phenyl group that may be substituted with an alkyl group that may be deuterated or a deuterium atom. In one aspect of the invention, X is an oxygen atom. In one aspect of the invention, X is a sulfur atom.

Specific examples of the benzofurocarbazole-9-yl group and the benzothienocarbazole-9-yl group in the formula (1a), the bisbenzofurocarbazole-9-yl group and the bisbenzothienocarbazole-9-yl group in the formula (1b), and substitutes thereof are given below. Here, a structure that can be adopted in the invention is not construed as limiting to such specific examples. * represents a bond position to the meta-phenylene in the formula (1a) and the formula (1b). Ph represents an unsubstituted phenyl group. Further, in this application, for a methyl group, the indication of CH₃ is omitted without indicating as CH₃. For example, D7 has a methyl group.

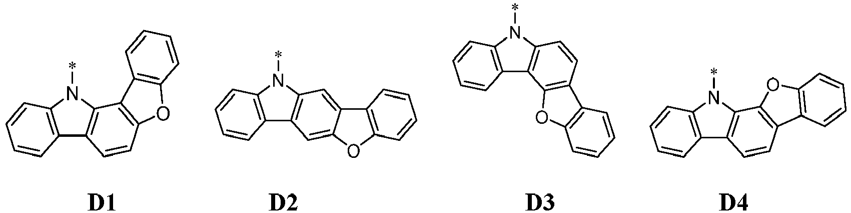

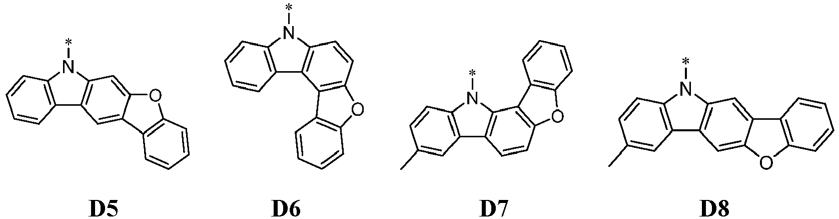

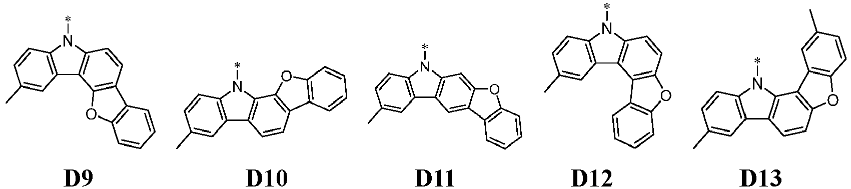

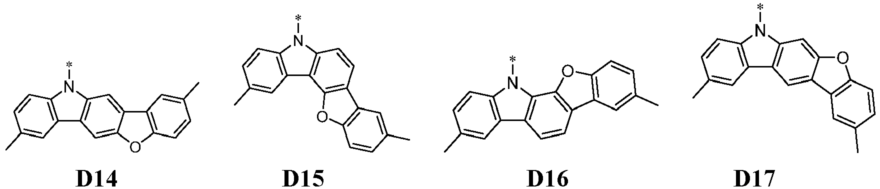

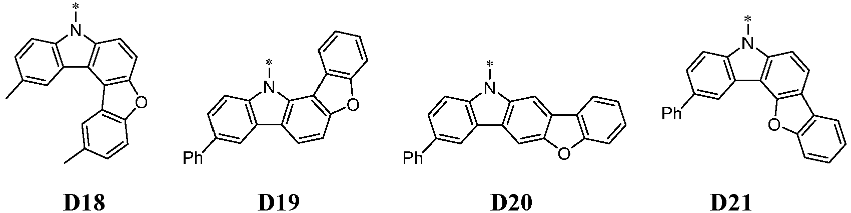

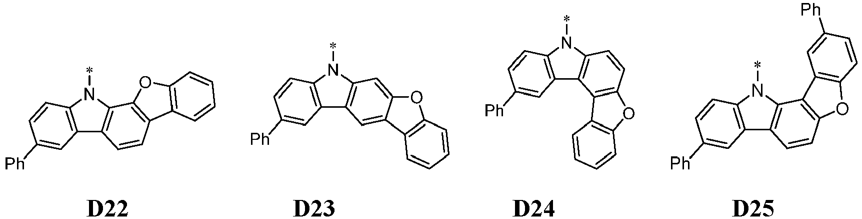

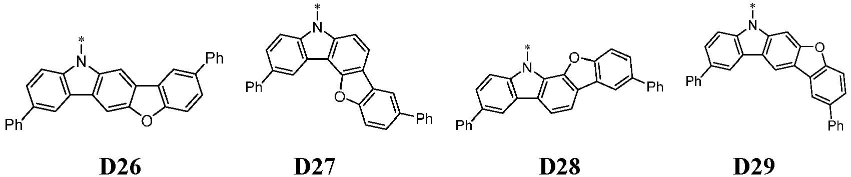

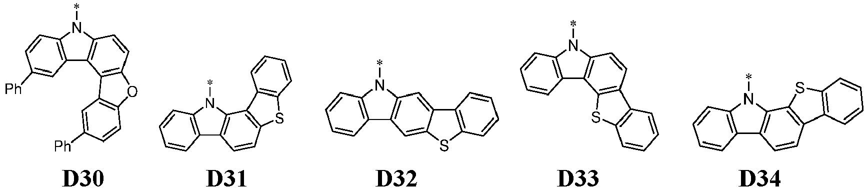

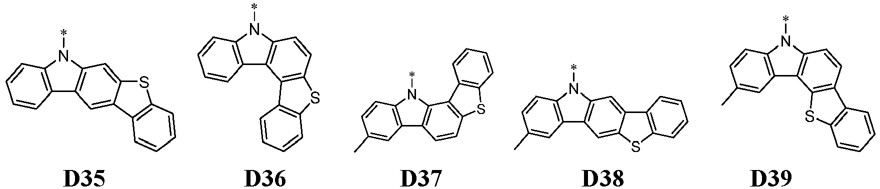

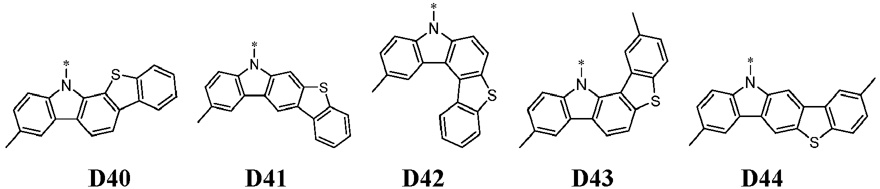

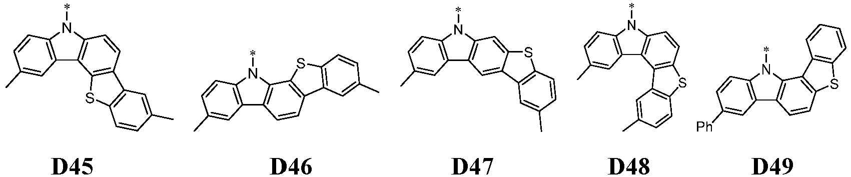

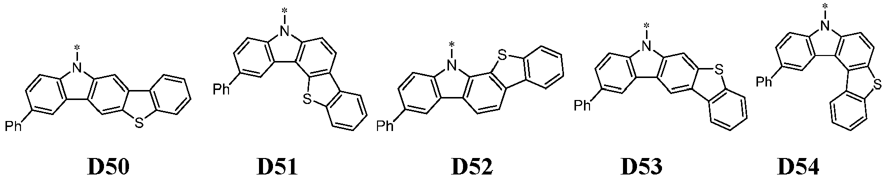

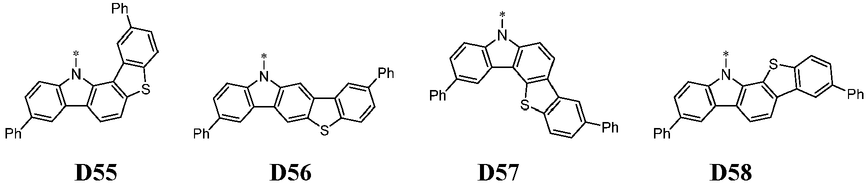

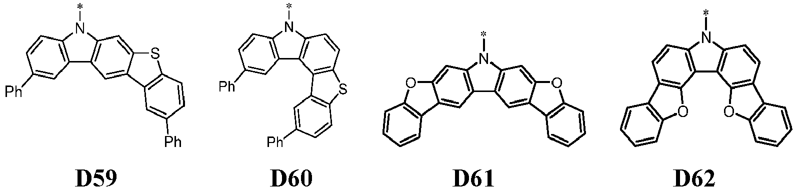

D7 to D18 and D37 to D48 in which all of hydrogen atoms present in the methyl group are substituted with a deuterium atom are disclosed herein as D7(d) to D18(d) and D37(d) to D48(d), respectively. Further, D19 to D30 and D49 to D60 in which all of hydrogen atoms present in the phenyl group are substituted with a deuterium atom are disclosed herein as D19(d) to D30(d) and D49(d) to D60(d), respectively. Further, D1 to D62 in which all of hydrogen atoms are substituted with a deuterium atom are disclosed herein as D1(D) to D62(D), respectively.

The dibenzofuran indicated on the left side of the formula (1) may be bonded to the meta-phenylene group at any of 1 to 4 positions. In a preferred aspect of the invention, the dibenzofuran is bonded to the meta-phenylene group at the 2-position. In one aspect of the invention, the dibenzofuran is bonded to the meta-phenylene group at the 1-position. In one aspect of the invention, the dibenzofuran is bonded to the meta-phenylene group at the 3-position. In one aspect of the invention, the dibenzofuran is bonded to the meta-phenylene group at the 4-position. The 1 to 4 positions of the dibenzofuran are as follows.

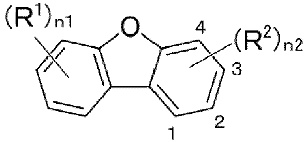

In the formula (1), there is no cyano group. In a preferred aspect of the invention, the formula (1) includes only a carbon atom, a hydrogen atom, a nitrogen atom, an oxygen atom, and a sulfur atom. In a preferred aspect of the invention, the formula (1) includes only a carbon atom, a hydrogen atom, a nitrogen atom, and an oxygen atom. The molecular weight of the compound represented by formula (1) is 499 or more, is preferably 800 or less, more preferably 700 or less, and still further preferably 600 or less, and for example, may be 550 or less, or may be 530 or less.

As the compound represented by the formula (1), a compound represented by the following formula (2) can be preferably exemplified.

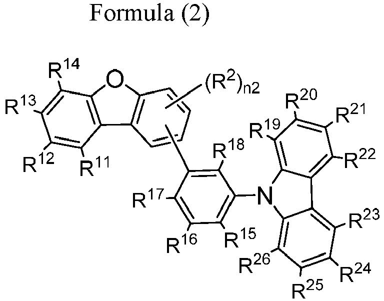

In the formula (2), R² represents an alkyl group that may be deuterated, and n2 represents an integer of 0 to 3. Each of R¹¹ to R¹⁸ independently represents a hydrogen atom, a deuterium atom, or an alkyl group that may be deuterated. Each of R¹⁹ to R²⁶ independently represents a hydrogen atom, a deuterium atom, an alkyl group that may be deuterated, or a phenyl group that may be substituted with an alkyl group that may be deuterated or a deuterium atom. Here, one or two among R¹⁹ to R²⁶ are a phenyl group that may be substituted with an alkyl group that may be deuterated or a deuterium atom. R¹⁹ and R²⁰, R²⁰ and R²¹, R²¹ and R²², R²³ and R²⁴, R²⁴ and R²⁵, and R²⁵ and R²⁶ may be bonded to each other to form a benzofuro skeleton or a benzothieno skeleton.

In a preferred aspect of the invention, at least one of R²¹ and R²⁴ is a phenyl group that may be substituted with an alkyl group that may be deuterated or a deuterium atom. In a preferred aspect of the invention, both of R²¹ and R²⁴ are a phenyl group that may be substituted with an alkyl group that may be deuterated or a deuterium atom. For example, at least one of R²¹ and R²⁴ is an unsubstituted phenyl group. For example, both of R²¹ and R²⁴ are an unsubstituted phenyl group. In one aspect of the invention, none of R¹⁹ and R²⁰, R²⁰ and R²¹, R²¹ and R²², R²³ and R²⁴, R²⁴ and R²⁵ , and R²⁵ and R²⁶ are bonded to each other to form a ring structure. In one aspect of the invention, in only one set among R¹⁹ and R²⁰, R²⁰ and R²¹, R²¹ and R²², R²³ and R²⁴, R²⁴ and R²⁵, and R²⁵ and R²⁶, these are bonded to each other to form a benzofuro skeleton or a benzothieno skeleton. In one aspect of the invention, in only one set among R¹⁹ and R²⁰, R²⁰ and R²¹, and R²¹ and R²², and only one set among R²³ and R²⁴, R²⁴ and R²⁵, and R²⁵ and R²⁶, these are bonded to each other to form a benzofuro skeleton or a benzothieno skeleton. In one aspect of the invention, R¹² represents an alkyl group that may be deuterated.

Hereinafter, specific examples of the compound represented by the formula (1) will be given, but a compound that can be used in the invention is not construed as limiting to such specific examples. For each of the following structures, four types of compounds are specified in accordance with the bond position of the dibenzofuran.

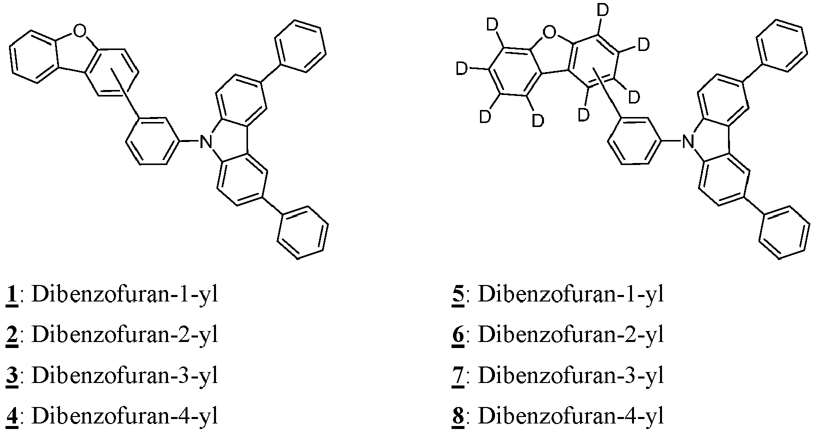

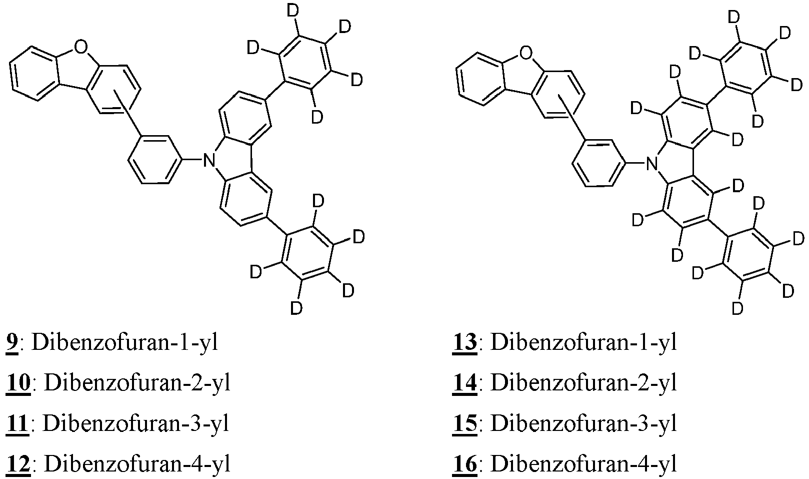

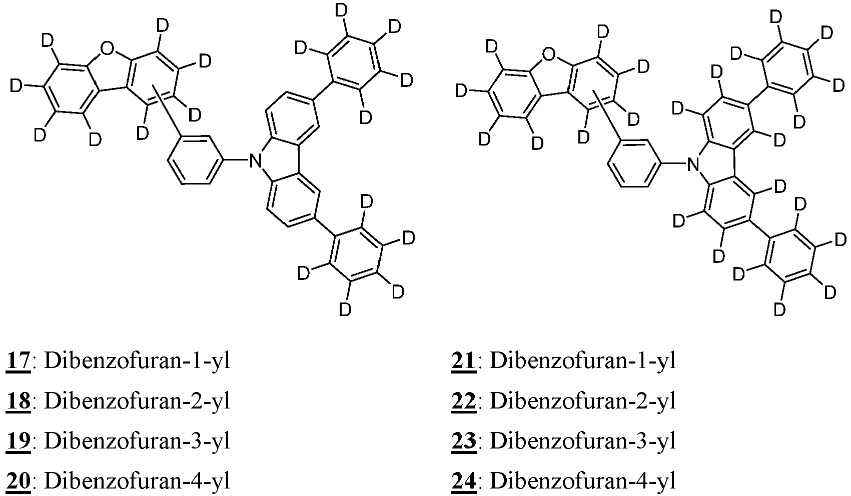

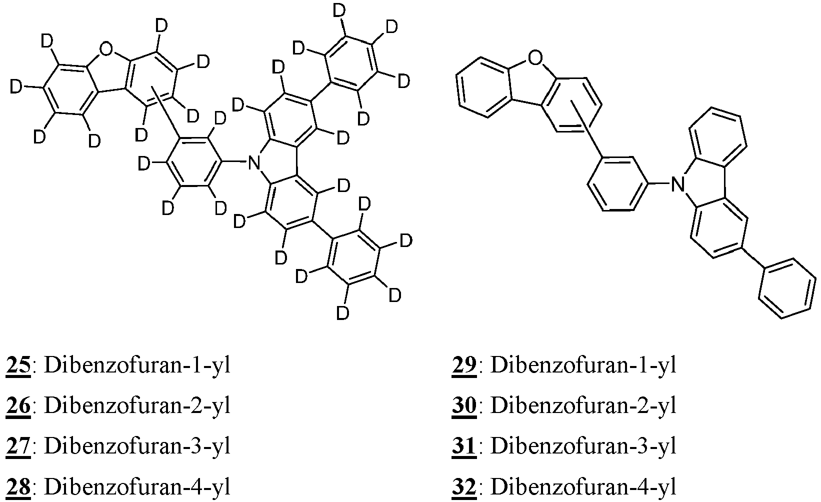

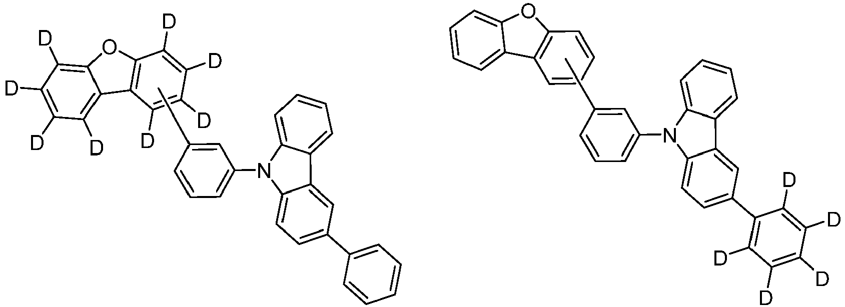

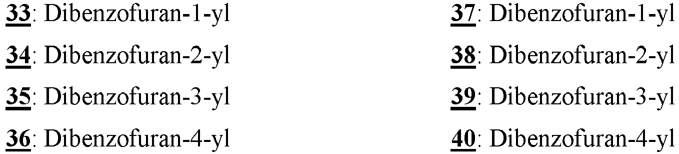

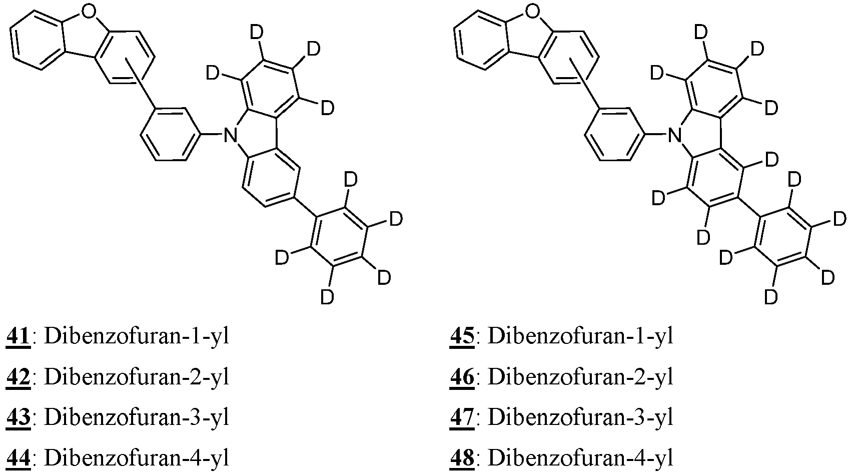

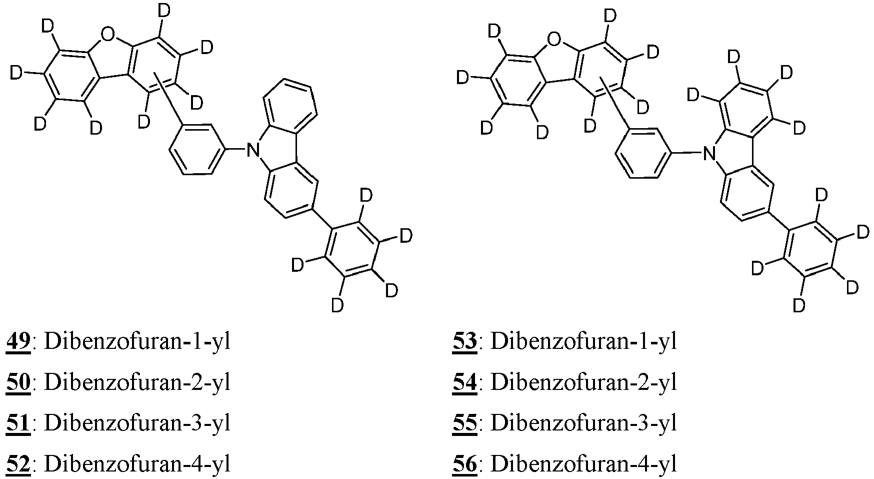

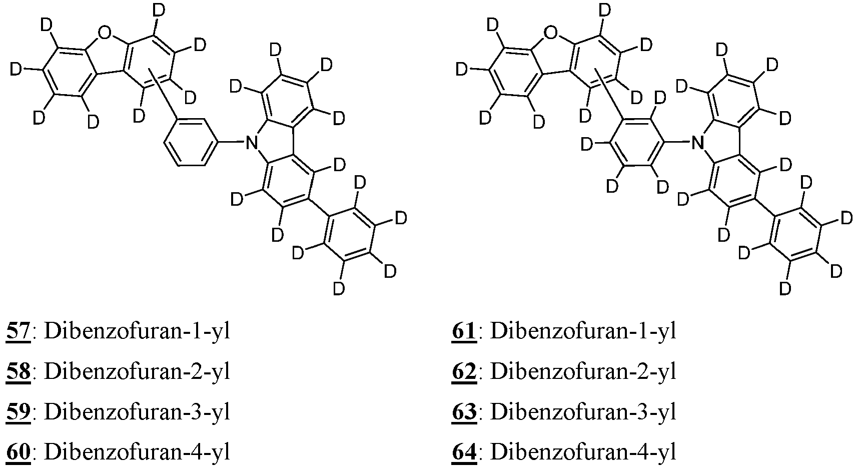

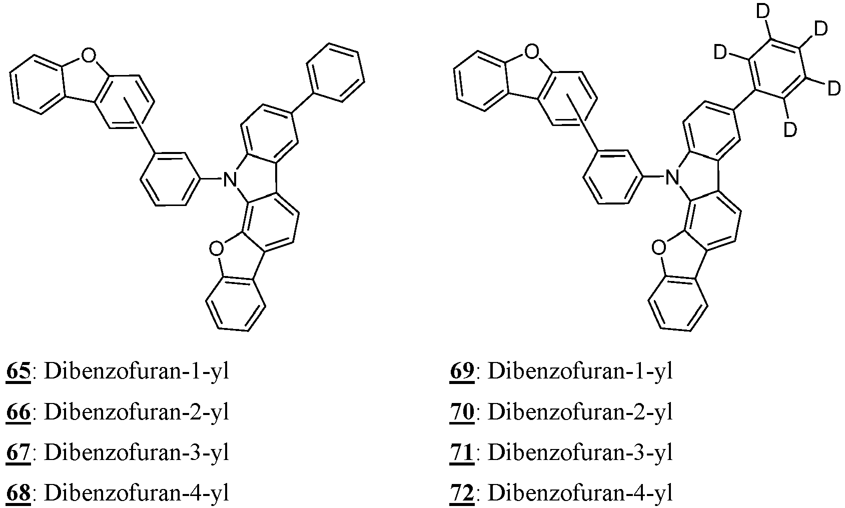

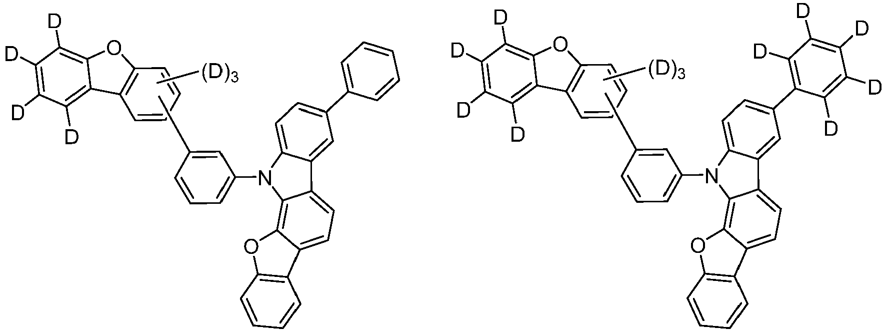

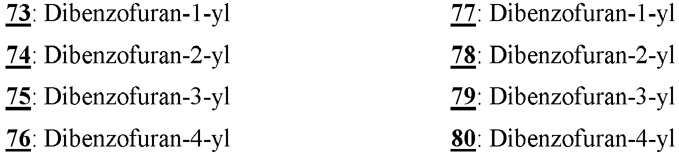

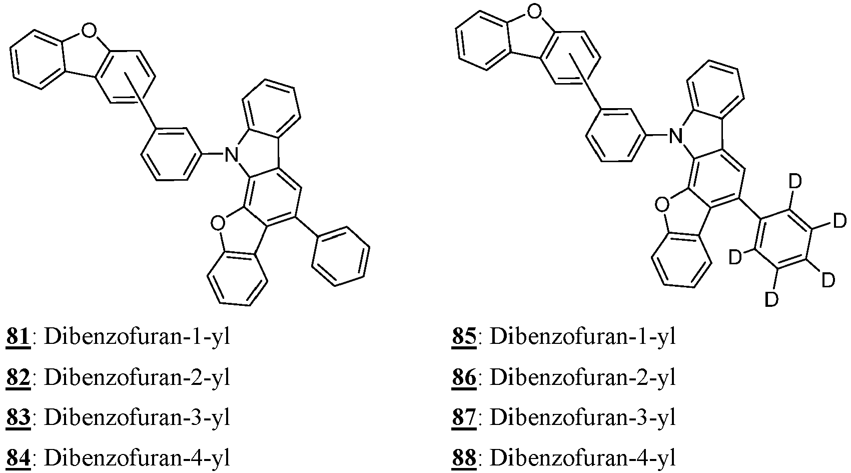

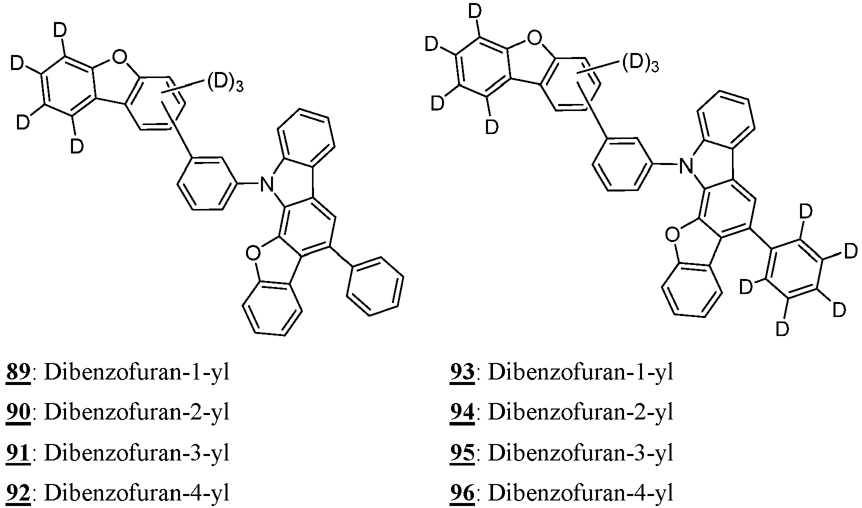

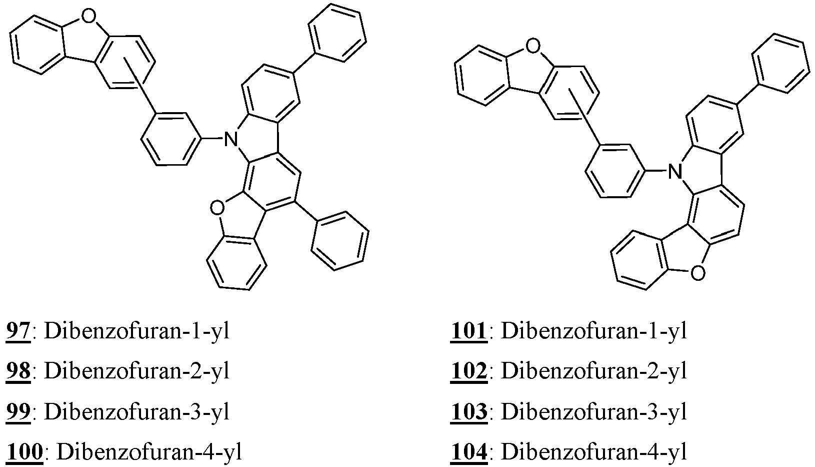

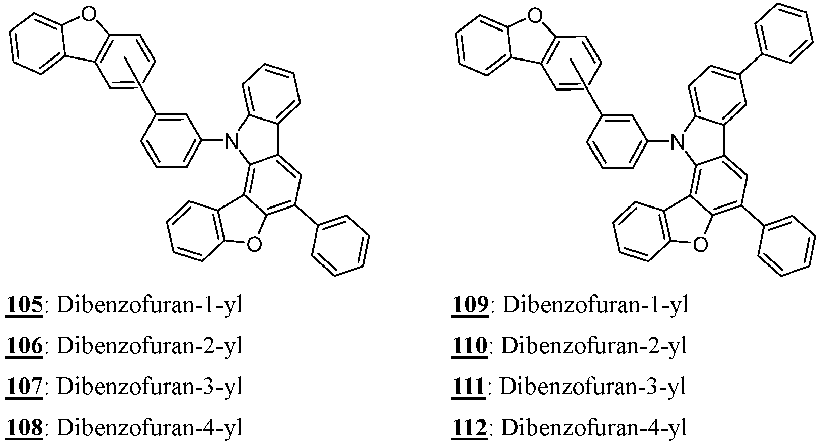

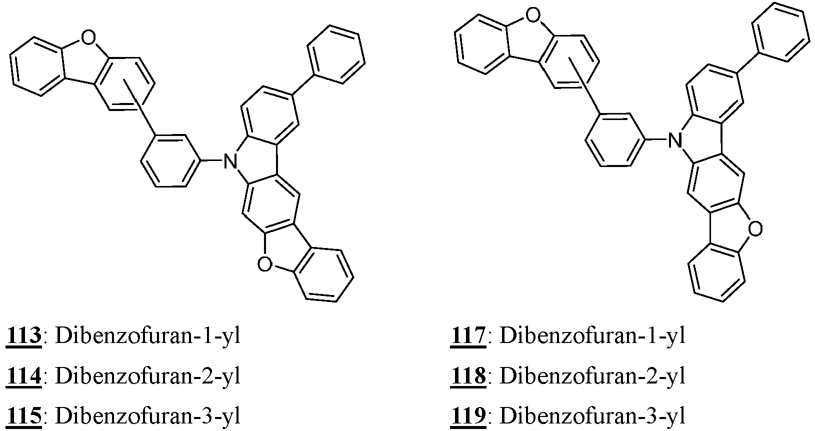

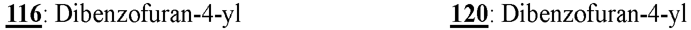

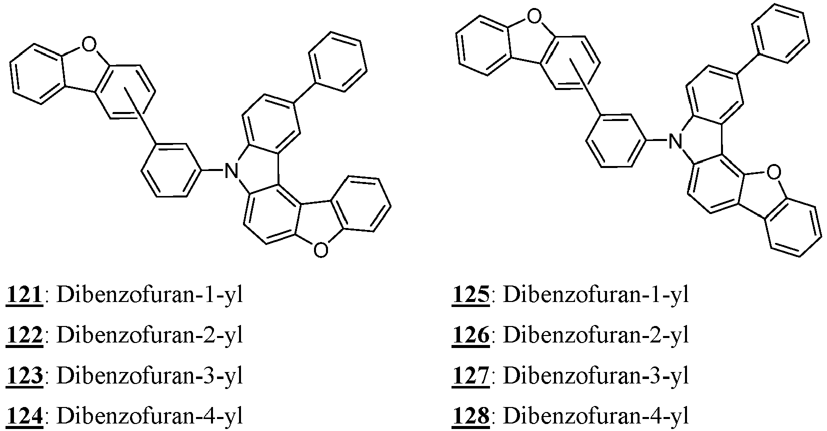

In one aspect of the invention, as the compound represented by the formula (1), any of the compounds 1 to 64 can be selected. In one aspect of the invention, as the compound represented by the formula (1), any of the compounds 65 to 128 can be selected.

The compound represented by the formula (1) is useful as a host material for doping a light-emitting material. In particular, the compound is useful as a host material for doping a delayed fluorescence material. There may be only one type of doping material, or a plurality of types of doping materials. The doping material is selected from materials having lowest excited singlet energy lower than that of the compound represented by the formula (1).

The compound represented by the formula (1) is also useful as an electron barrier material. In particular, the compound is useful as an electron barrier material in an organic light-emitting device using a delayed fluorescence material. For example, in the organic light-emitting device such as an organic electroluminescence device, the compound can be effectively used in an electron barrier layer. In particular, the compound can be effectively used in an electron barrier layer adjacent to a light-emitting layer using a delayed fluorescence material.

Examples of a similar compound of the formula (1) include a compound represented by the following formula (3) in which a meta-phenylene linking structure in the formula (1) is substituted with a biphenylene linking structure. The compound represented by the formula (3) is also useful as a host material, and useful as an electron barrier material, but the compound represented by the formula (1) is more effective and more useful than the compound represented by the formula (3), as the host material or the electron barrier material. For the description on R¹, R², R⁴, R⁵, n1, n2, n4, and n5 in the following formula (3), corresponding description on the formula (1) can be referred to. For R^{3a} and R^{3b} in the formula (3), the description of R³ in the formula (1) can be referred to. Further, specific examples of the formula (3) include a formula in which the phenylene linking structure is substituted with the biphenylene linking structure in the specific example of the formula (1).

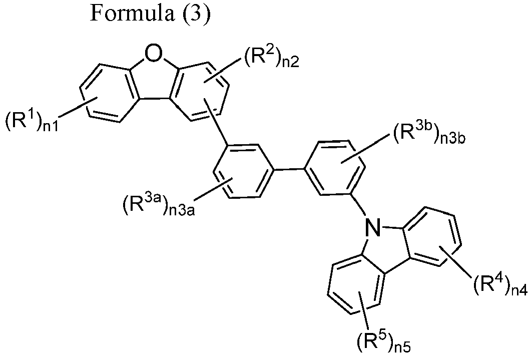

### (Delayed fluorescence material)

The compound represented by the formula (1) is useful as the host material for use together with the delayed fluorescence material.

The "delayed fluorescence material" mentioned herein is an organic compound that causes inverse intersystem crossing to the excited singlet state from the excited triplet state in the excited state, and then, emits delayed fluorescence when returning to the ground state from the excited singlet state. In the invention, when the emission lifetime is measured by a fluorescence lifetime measurement system (available from Hamamatsu Photonics K. K., a streak camera system or the like), a material in which fluorescence having emission lifetime of 100 ns (nanoseconds) or more is observed is referred to as a delayed fluorescence material.

When the compound represented by the formula (1) is used in combination with the delayed fluorescence material, the delayed fluorescence material receives the energy from the compound represented by the formula (1) in the excited singlet state to transition to the excited singlet state. Further, the delayed fluorescence material may receive the energy from the compound represented by the formula (1) in the excited triplet state to transition to the excited triplet state. Since the delayed fluorescence material has a small difference (AE_{ST}) between the excited singlet energy and the excited triplet energy, inverse intersystem crossing to the delayed fluorescence material in the excited singlet state from the delayed fluorescence material in the excited triplet state is likely to occur. The delayed fluorescence material in the excited singlet state generated through such a route contributes to light emission.

In the delayed fluorescence material, a difference ΔE_{ST} between the lowest excited singlet energy and the lowest excited triplet energy of 77 K is preferably 0.3 eV or less, more preferably 0.25 eV or less, more preferably 0.2 eV or less, more preferably 0.15 eV or less, further preferably 0.1 eV or less, still further preferably 0.07 eV or less, still further preferably 0.05 eV or less, still further preferably 0.03 eV or less, and particularly preferably 0.01 eV or less.

When ΔE_{ST} is small, inverse intersystem crossing to the excited triplet state from the excited singlet state is likely to occur through the absorption of the thermal energy, and thus, the delayed fluorescence material functions as a thermally activated delayed fluorescence material. The thermally activated delayed fluorescence material absorbs the heat emitted from the device to relatively easily cause the inverse intersystem crossing to the excited singlet state from the excited triplet state, which may contribute to efficient emission of the excited triplet energy.

In the invention, the lowest excited singlet energy (E_{S1}) and the lowest excited triplet energy (E_{T1}) of the compound are a value obtained by the following procedure. ΔE_{ST} is a value obtained by calculating E_{S1}-E_{T1}.

### (1) Lowest excited singlet energy (E_{S1})

A thin film or a toluene solution (a concentration of 10⁻⁵ mol/L) of a measurement target compound is prepared as a specimen. The fluorescent spectrum of the specimen is measured at room temperature (300 K). In the fluorescent spectrum, a vertical axis is set as light emission, and a horizontal axis is set as a wavelength. A tangential line is drawn to the rise of the emission spectrum on the short wavelength side, and a wavelength value λedge [nm] at an intersection between the tangential line and the horizontal axis is obtained. A value obtained by converting the wavelength value into an energy value through the following conversion formula is set as E_{S1}.

### Conversion Formula: E_{S1}[eV]=1239.85/λedge

The emission spectrum in Examples described below was measured by a detector (available from Hamamatsu Photonics K.K., PMA-12 multichannel spectroscope C10027-01) using an LED light source (available from Thorlabs, Inc., M300L4) as an excitation light source.

### (2) Lowest excited triplet energy (E_{T1})

The same specimen as that used for the measurement of the lowest excited singlet energy (E_{S1}) is cooled to 77 [K] by liquid nitrogen, and a specimen for measuring phosphorescence is irradiated with excitation light (300 nm) to measure the phosphorescence with a detector. Emission after 100 milliseconds after the irradiation of the excitation light is set as a phosphorescence spectrum. A tangential line is drawn to the rise of the phosphorescent spectrum on the short wavelength side, and a wavelength value λedge [nm] at an intersection between the tangential line and the horizontal axis is obtained. A value obtained by converting the wavelength value into an energy value through the following conversion formula is set as E_{T1}.

### Conversion Formula: E_{T1}[eV]=1239.85/λedge

The tangential line to the rise of the phosphorescent spectrum on the short wavelength side is drawn as follows. When moving on a spectrum curve from the short wavelength side of the phosphorescence spectrum to the maximum value on the shortest wavelength side among the maximum values of the spectrum, a tangential line at each point on the curve toward the long wavelength side is taken into consideration. The slope of the tangential line increases as the curve rises (that is, as the vertical axis increases). A tangential line drawn at a point where the value of the slope is the maximum value is set as the tangential line to the rise of the phosphorescent spectrum on the short wavelength side.

The maximum point with a peak intensity of 10% or less of the largest peak intensity of the spectrum is not included in the maximum value on the shortest wavelength side, but is closest to the maximum value on the shortest wavelength side, and a tangential line drawn at a point where the value of the slope is the maximum value is set as the tangential line to the rise of the phosphorescent spectrum on the short wavelength side.

In a preferred aspect of the invention, as the delayed fluorescence material, a compound (cyanobenzene derivative) having a cyanobenzene structure in which the number of cyano groups substituted with the benzene ring is 1 is used. In another preferred aspect of the invention, as the delayed fluorescence material, a compound (dicyanobenzene derivative) having a dicyanobenzene structure in which the number of cyano groups substituted with the benzene ring is 2 is used. In another preferred aspect of the invention, as the delayed fluorescence material, a compound (azabenzene derivative) having an azabenzene structure in which at least one of ring skeleton forming carbon atoms of a benzene ring is substituted with a nitrogen atom is used.

In a preferred aspect of the invention, as the delayed fluorescence material, a compound represented by the following formula (4) is used.

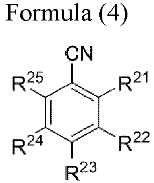

In the formula (4), one among R²¹ to R²³ represents a cyano group or a group represented by the following formula (5), the rest two of R²¹ to R²³ and at least one of R²⁴ and R²⁵ represent a group represented by the following formula (6), and the rest of R²¹ to R²⁵ represents a hydrogen atom or a substituent (the substituent mentioned herein is not the cyano group, the group represented by the following formula (5), and the group represented by the following formula (6)).

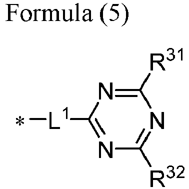

In the formula (5), L¹ represents a single bond or a divalent linking group, each of R³¹ and R³² independently represents a hydrogen atom or a substituent, and * represents a bond position.

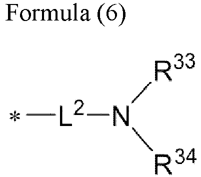

In the formula (6), L² represents a single bond or a divalent linking group, each of R³³ and R³⁴ independently represents a hydrogen atom or a substituent, and * represents a bond position.

In a preferred aspect of the invention, R²² is a cyano group. In a preferred aspect of the invention, R²² is the group represented by the formula (5). In one aspect of the invention, R²¹ is a cyano group or the group represented by the formula (5). In one aspect of the invention, R²³ is a cyano group or the group represented by the formula (5). In one aspect of the invention, one of R²¹ to R²³ is a cyano group. In one aspect of the invention, one among R²¹ to R²¹ is the group represented by the formula (5).

In a preferred aspect of the invention, L¹ in the formula (5) is a single bond. In one aspect of the invention, L¹ is a divalent linking group, preferably a substituted or unsubstituted arylene group, or a substituted or unsubstituted heteroarylene group, more preferably a substituted or unsubstituted arylene group, and further preferably a substituted or unsubstituted 1,4-phenylene group (examples of the substituent include an alkyl group having 1 to 3 carbon atoms).

In one aspect of the invention, each of R³¹ and R³² in the formula (5) independently represents one group selected from the group consisting of an alkyl group (e.g., 1 to 40 carbon atoms), an aryl group (e.g., 6 to 30 carbon atoms), a heteroaryl group (e.g., 5 to 30 ring skeletons forming atoms), an alkenyl group (e.g., 1 to 40 carbon atoms) and an alkynyl group (e.g., 1 to 40 carbon atoms), or a group formed by combining two or more thereof (hereinafter, such groups will be referred to as a "group of a substituent group A"). In a preferred aspect of the invention, each of R³¹ and R³² independently represents a substituted or unsubstituted aryl group (e.g., 6 to 30 carbon atoms), and examples of the substituent of the aryl group include the groups of the substituent group A. In a preferred aspect of the invention, R³¹ and R³² are the same.

In a preferred aspect of the invention, L² in the formula (6) is a single bond. In one aspect of the invention, L² is a divalent linking group, preferably is a substituted or unsubstituted arylene group, or a substituted or unsubstituted heteroarylene group, more preferably a substituted or unsubstituted arylene group, and further preferably a substituted or unsubstituted 1,4-phenylene group (examples of the substituent include an alkyl group having 1 to 3 carbon atoms).

In one aspect of the invention, each of R³³ and R³⁴ in the formula (6) independently represents a substituted or unsubstituted alkyl group (e.g., 1 to 40 carbon atoms), a substituted or unsubstituted alkenyl group (e.g., 1 to 40 carbon atoms), a substituted or unsubstituted aryl group (e.g., 6 to 30 carbon atoms), or a substituted or unsubstituted heteroaryl group (e.g., 5 to 30 carbon atoms). Examples of the substituents of the alkyl group, the alkenyl group, the aryl group, and the heteroaryl group mentioned herein include one group selected from the group consisting of a hydroxy group, a halogen atom (e.g., a fluorine atom, a chlorine atom, a bromine atom, an iodine atom), an alkyl group (e.g., 1 to 40 carbon atoms), an alkoxy group (e.g., 1 to 40 carbon atoms), an alkylthio group (e.g., 1 to 40 carbon atoms), an aryl group (e.g., 6 to 30 carbon atoms), an aryl oxy group (e.g., 6 to 30 carbon atoms), an arylthio group (e.g., 6 to 30 carbon atoms), a heteroaryl group (e.g., 5 to 30 ring skeletons forming atoms), a heteroaryl oxy group (e.g., 5 to 30 ring skeletons forming atoms), a heteroarylthio group (e.g., 5 to 30 ring skeletons forming atoms), an acyl group (e.g., 1 to 40 carbon atoms), an alkenyl group (e.g., 1 to 40 carbon atoms), an alkynyl group (e.g., 1 to 40 carbon atoms), an alkoxycarbonyl group (e.g., 1 to 40 carbon atoms), an aryl oxycarbonyl group (e.g., 1 to 40 carbon atoms), a heteroaryl oxycarbonyl group (e.g., 1 to 40 carbon atoms), a silyl group (e.g., a trialkyl silyl group having 1 to 40 carbon atoms), a nitro group and a cyano group, or a group formed by combining two or more thereof (hereinafter, such groups will be referred to as a "group of the substituent group B")

R³³ and R³⁴ may be bonded to each other via a single bond or a linking group to form a ring structure. In particular, in a case where R³³ and R³⁴ are an aryl group, it is preferable that R³³ and R³⁴ are bonded to each other via a single bond or a linking group to form a ring structure. Examples of the linking group mentioned herein include -O-, - S-, -N(R³⁵)-, -C(R³⁶)(R³⁷)-, and -C(=O)-, and -O-, -S-, -N(R³⁵)-, and -C(R³⁶)(R³⁷)- are preferable, and -O-, -S-, and -N(R³⁵)- are more preferable. Each of R³⁵ to R³⁷ independently represents a hydrogen atom or a substituent. As the substituent, the group of the substituent group A can be selected, or the group of the substituent group B can be selected, and preferably, one group selected from the group consisting of an alkyl group having 1 to 10 carbon atoms and an aryl group having 6 to 14 carbon atoms, or a group formed by combining two or more thereof.

The group represented by the formula (6) is preferably a group represented by the following formula (7).

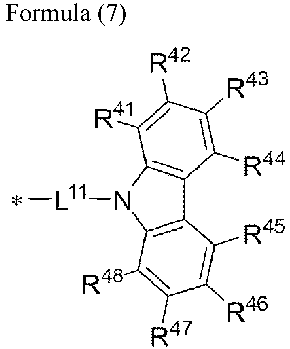

L¹¹ in the formula (7) represents a single bond or a divalent linking group. For the description and the preferable range of L¹¹, the description and the preferable range of L² can be referred to.

Each of R⁴¹ to R⁴⁸ in the formula (7) independently represents a hydrogen atom or a substituent. R⁴¹ and R⁴² , R⁴² and R⁴³, R⁴³ and R⁴⁴, R⁴⁴ and R⁴⁵, R⁴⁵ and R⁴⁶, R⁴⁶ and R⁴⁷, and R⁴⁷ and R⁴⁸ may be bonded to each other to form ring structures. The ring structure formed by the bonding may be an aromatic ring or an adipose ring, and may be a ring having hetero atoms, further, the ring structure may be a condensed ring of two or more rings. The hetero atom mentioned herein is preferably one selected from the group consisting of a nitrogen atom, an oxygen atom, and a sulfur atom. Examples of the ring structure to be formed include a benzene ring, a naphthalene ring, a pyridine ring, a pyridazine ring, a pyrimidine ring, a pyrazine ring, a pyrrole ring, an imidazole ring, a pyrazole ring, an imidazoline ring, an oxazole ring, an isooxazole ring, a thiazole ring, an isothiazole ring, a cyclohexadiene ring, a cyclohexene ring, a cyclopentaene ring, a cycloheptatriene ring, a cycloheptadiene ring, a cycloheptaene ring, a furan ring, a thiophene ring, a naphthyridine ring, a quinoxaline ring, and a quinoline ring. For example, a ring in which a plurality of rings are condensed, such as a phenanthrene ring or a triphenylene ring, may be formed. As a preferable example, a benzofuran ring or a benzothiophene ring can be exemplified (condensed with a furan ring and a thiophene ring). The number of rings included in the group represented by the formula (7) may be selected from a range of 3 to 5, or may be selected from a range of 5 to 7.

Examples of the substituent that may be possessed by R⁴¹ to R⁴⁸ include the group of the substituent group B, and preferably an unsubstituted alkyl group having 1 to 10 carbon atoms, or an aryl group having 6 to 10 carbon atoms that may be substituted with an unsubstituted alkyl group having 1 to 10 carbon atoms. In a preferred aspect of the invention, R⁴¹ to R⁴⁸ are a hydrogen atom or an unsubstituted alkyl group having 1 to 10 carbon atoms. In a preferred aspect of the invention, R⁴¹ to R⁴⁸ are a hydrogen atom or an unsubstituted aryl group having 6 to 10 carbon atoms. In a preferred aspect of the invention, all of R⁴¹ to R⁴⁸ are a hydrogen atom.

In a preferred aspect of the invention, two or more of R²¹ to R²⁵ in the formula (4) are the group represented by the formula (7), and all of the groups represented by the formula (7) are not the same but different from each other in at least one of R⁴¹ to R⁴⁸. In a preferred aspect of the invention, two or more of R²¹ to R²⁵ in the formula (4) are the group represented by the formula (7), and one of R²¹ to R²⁵ is a phenyl group that may be substituted with a deuterium atom or an alkyl group.

In the formula (7), * represents a bond position.

In a preferred aspect of the invention, as the delayed fluorescence material, an azabenzene derivative is used. In a preferred aspect of the invention, the azabenzene derivative has an azabenzene structure in which three ring skeletons forming carbon atoms of a benzene ring are substituted with a nitrogen atom. For example, an azabenzene derivative having a 1,3,5-triazine structure can be preferably selected. In a preferred aspect of the invention, the azabenzene derivative has an azabenzene structure in which two ring skeletons forming carbon atoms of a benzene ring are substituted with a nitrogen atom. For example, azabenzene derivatives having a pyridazine structure, a pyrimidine structure, and a pyrazine structure can be given, and the azabenzene derivative having a pyrimidine structure can be preferably selected. In one aspect of the invention, the azabenzene derivative has a pyridine structure in which one ring skeleton forming a carbon atom of a benzene ring is substituted with a nitrogen atom.

In a preferred aspect of the invention, as the delayed fluorescence material, a compound represented by the following formula (8) is used.

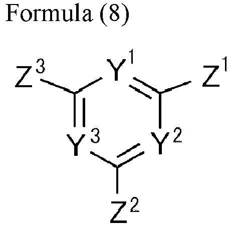

In the formula (8), at least one of Y¹, Y², and Y³ represents a nitrogen atom, and the rest represent a methine group. In one aspect of the invention, Y¹ is a nitrogen atom, and Y² and Y³ are a methine group. Preferably, Y¹ and Y² are a nitrogen atom, and Y³ is a methine group. More preferably, all of Y¹ to Y³ are a nitrogen atom.

In the formula (8), each of Z¹ to Z³ independently represents a hydrogen atom or a substituent, and at least one is a donor substituent. The donor substituent means a group having a negative Hammett σp value. Preferably, at least one of Z¹ to Z³ is a group having a diaryl amino structure (two aryl groups bonded to a nitrogen atom may be bonded to each other), more preferably, the group represented by the formula (6), and for example, the group represented by the formula (7). In one aspect of the invention, only one of Z¹ to Z³ is the group represented by the formula (6) or (7). In one aspect of the invention, each of only two of Z¹ to Z³ is independently the group represented by the formula (6) or (7). In one aspect of the invention, each of all of Z¹ to Z³ is independently the group represented by the formula (6) or (7). For the details and the preferable range of the formula (6) and the formula (7), the corresponding description can be referred to. It is preferable that the rest of Z¹ to Z³, which are not the groups represented by the formula (6) and the formula (7), are a substituted or unsubstituted aryl group (e.g., 6 to 40 carbon atoms, preferably 6 to 20), and as the substituent of the aryl group mentioned herein, one group selected from the group consisting of an aryl group (e.g., 6 to 20 carbon atoms, preferably 6 to 14) and an alkyl group (e.g., 1 to 20 carbon atoms, preferably 1 to 6), or a group formed by combining two or more thereof can be exemplified. In one aspect of the invention, the formula (8) does not include a cyano group.

In a preferred aspect of the invention, as the delayed fluorescence material, a compound represented by the following formula (9) is used.

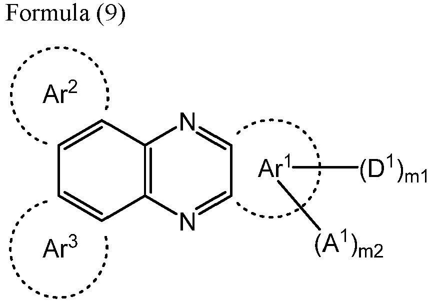

In the formula (9), Ar¹ forms a ring structure that may be substituted with A¹ and D¹ described below, and represents a benzene ring, a naphthalene ring, an anthracene ring, or a phenanthrene ring. Each of Ar² and Ar³ may form a ring structure, and in the case of forming the ring structure, represents a benzene ring, a naphthalene ring, a pyridine ring, or a benzene ring substituted with a cyano group. m1 represents any integer of 0 to 2, and m2 represents any integer of 0 and 1. A¹ represents a cyano group, a phenyl group, a pyrimidyl group, a triazyl group, or a benzonitrile group. D¹ represents a substituted or unsubstituted 5H-indolo[3,2,1-de]phenazine-5-yl group, or a substituted or unsubstituted hetero ring-condensed carbazolyl group not having a naphthalene structure, and in a case where there are a plurality of D¹'s in the formula (9), such D¹'s may be the same or different from each other. Further, the substituents of D¹ may be bonded to each other to form a ring structure.

Hereinafter, preferable compounds that can be used as the delayed fluorescence material will be given, but the delayed fluorescence material that can be used in the invention is not construed as limiting to such specific examples.

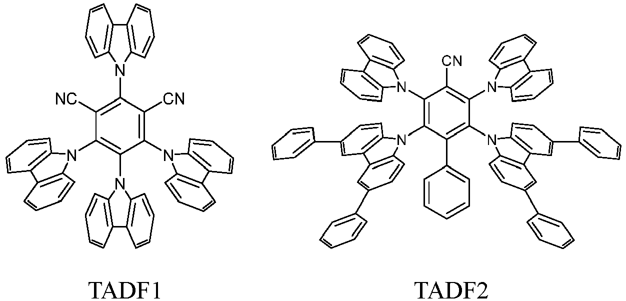

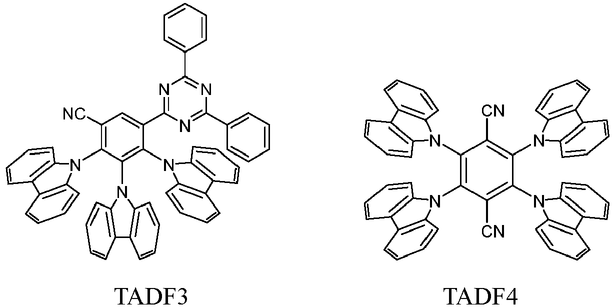

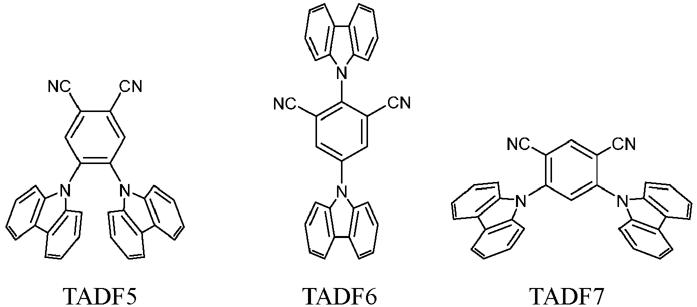

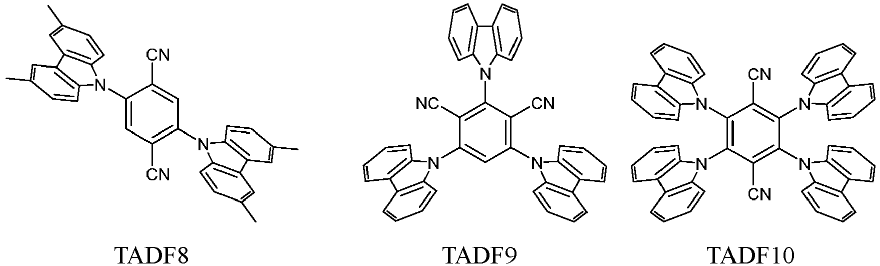

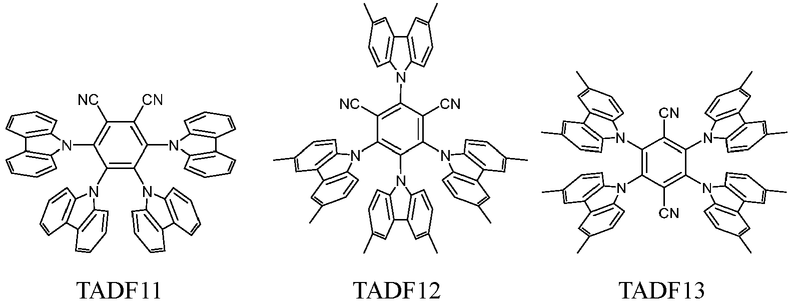

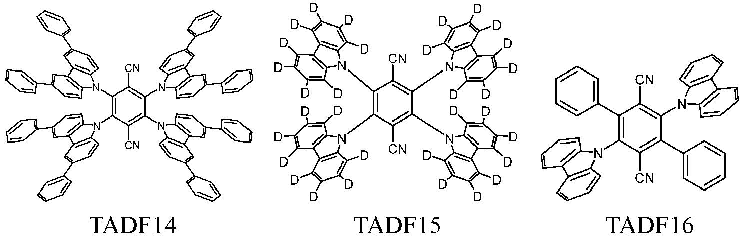

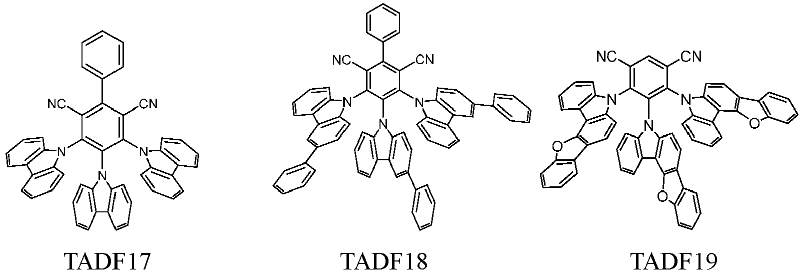

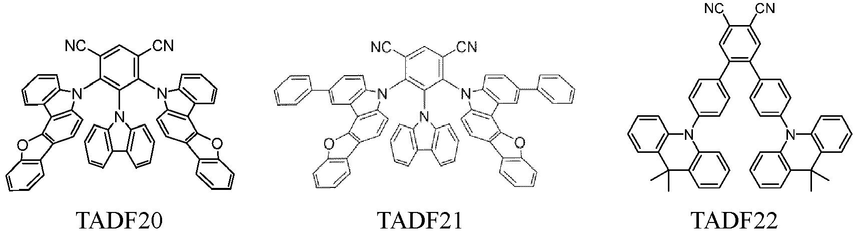

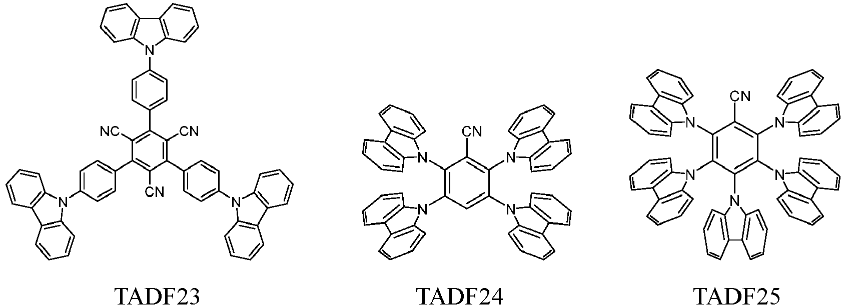

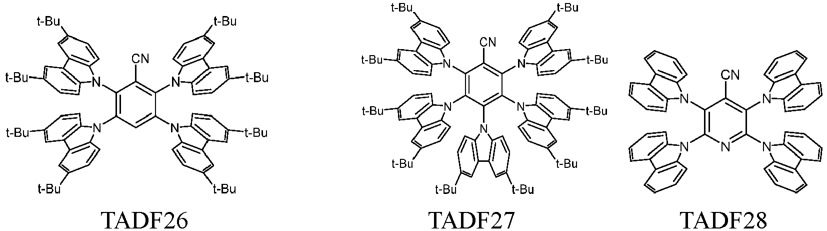

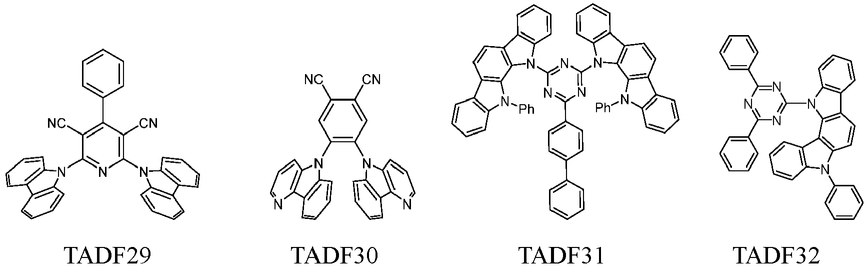

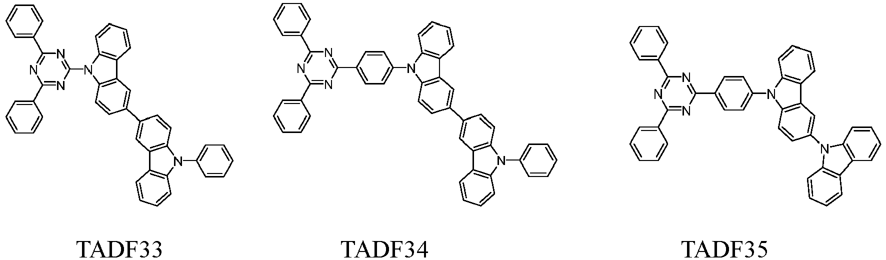

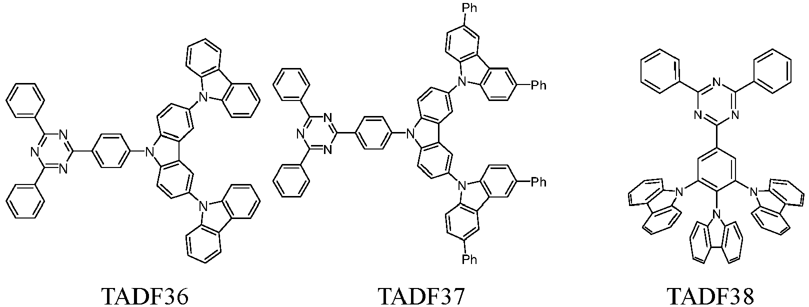

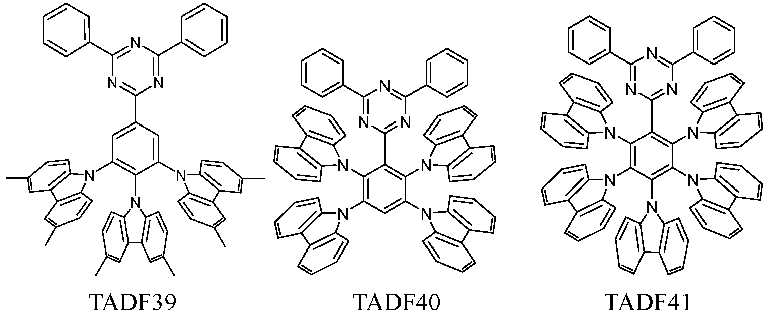

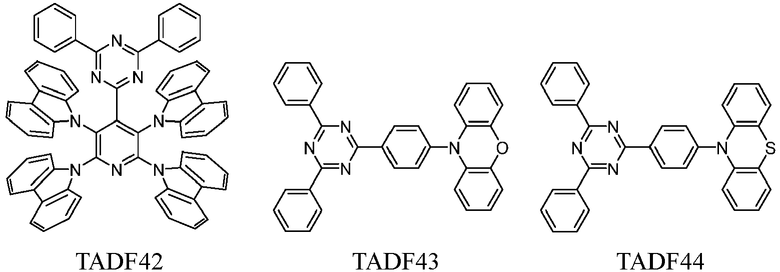

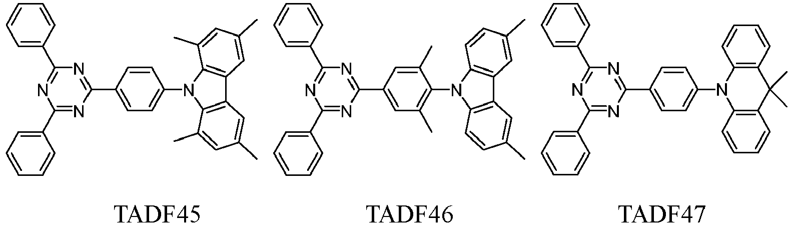

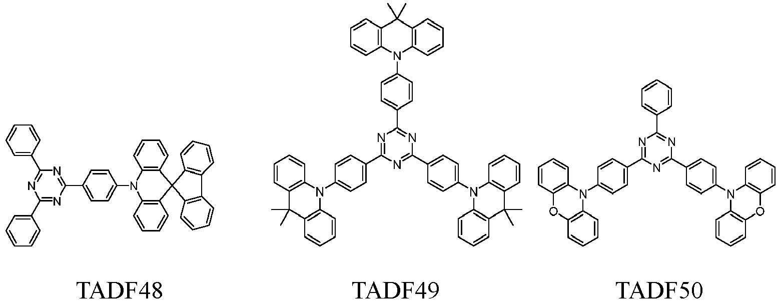

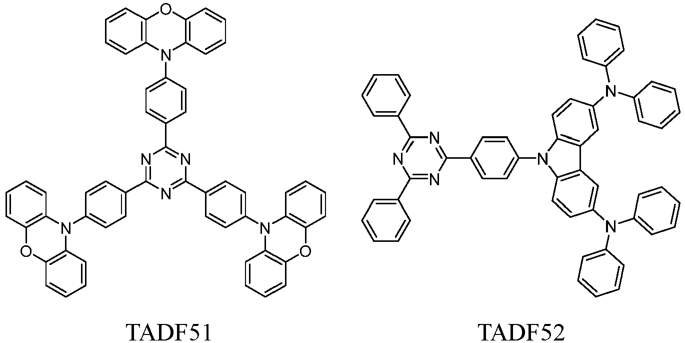

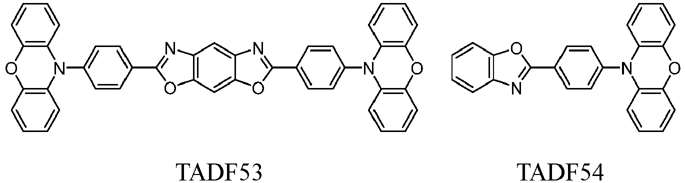

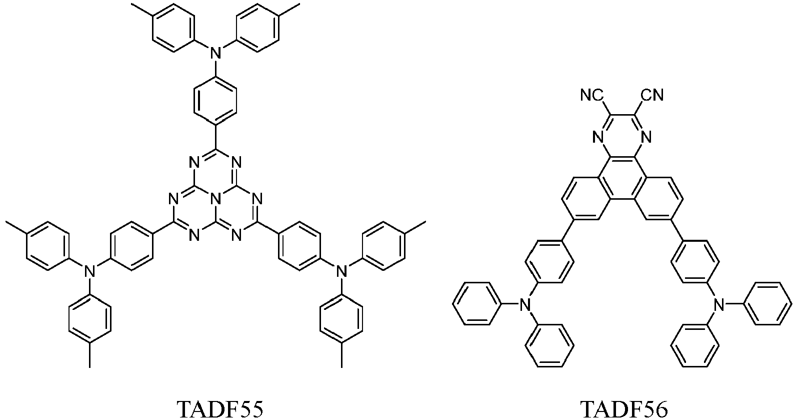

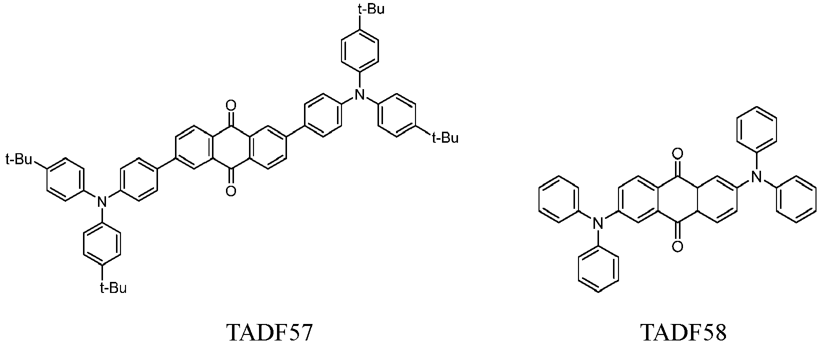

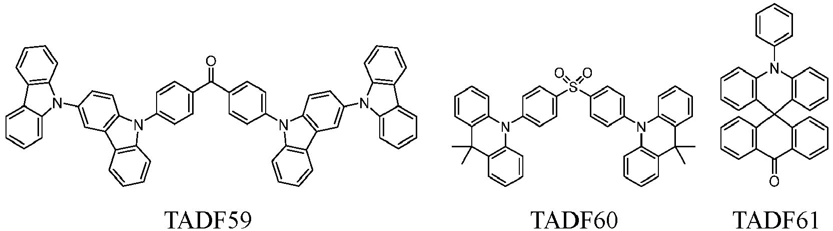

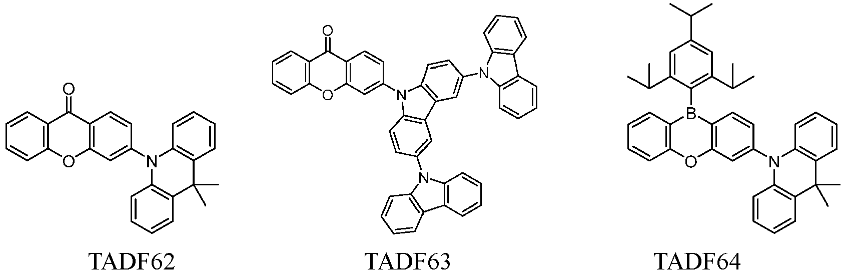

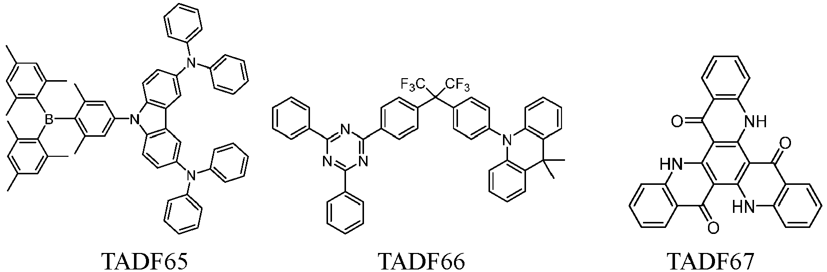

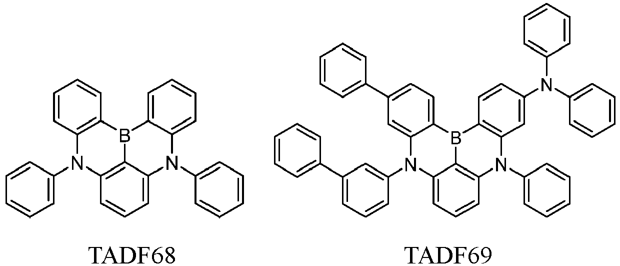

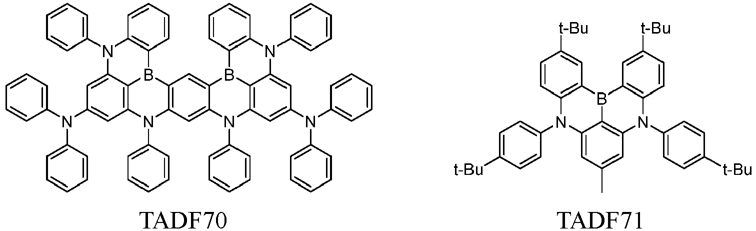

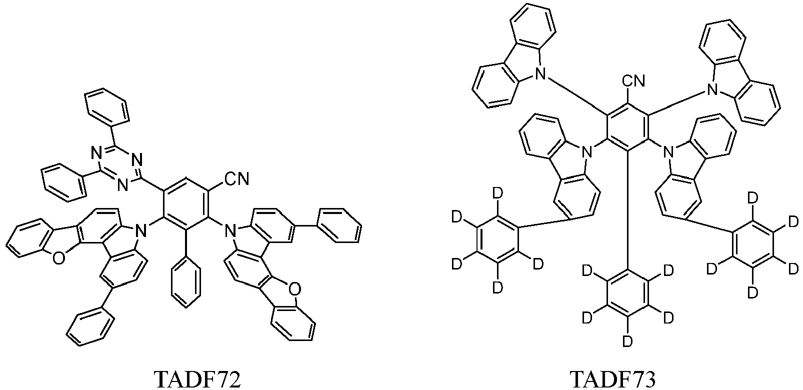

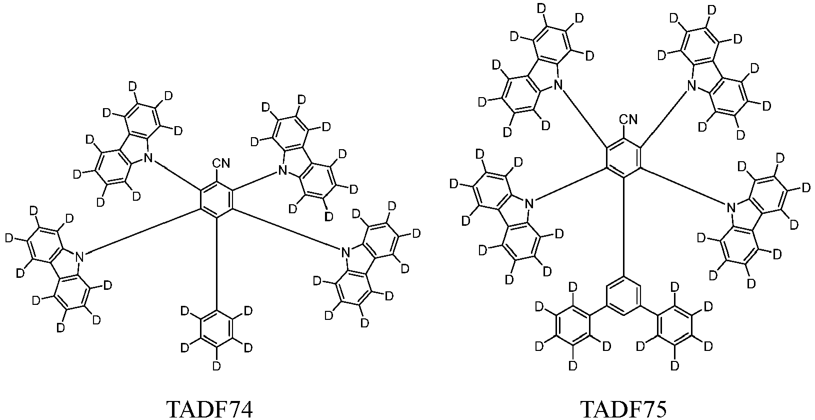

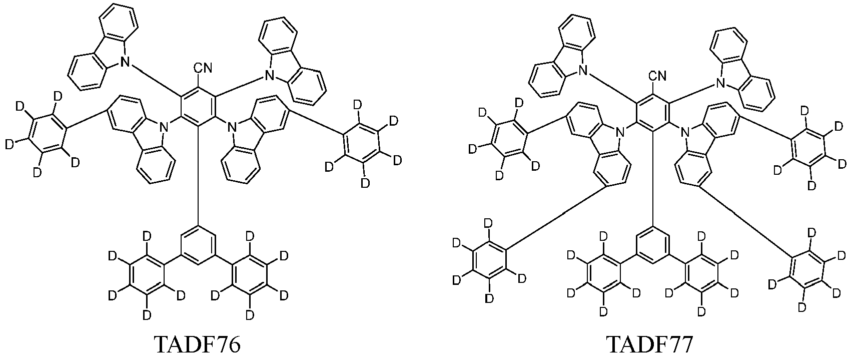

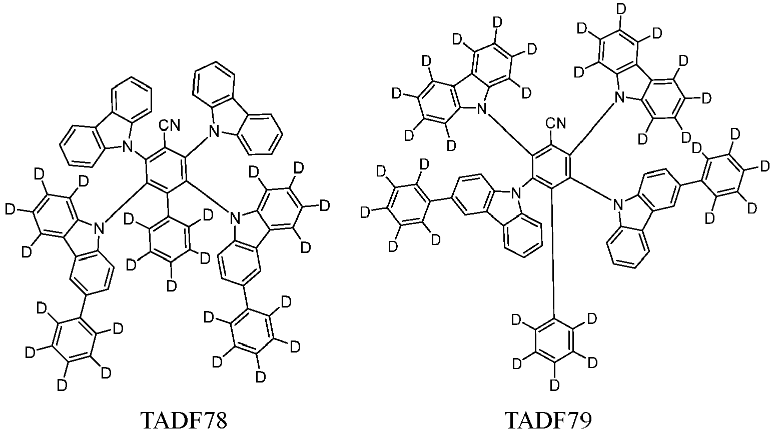

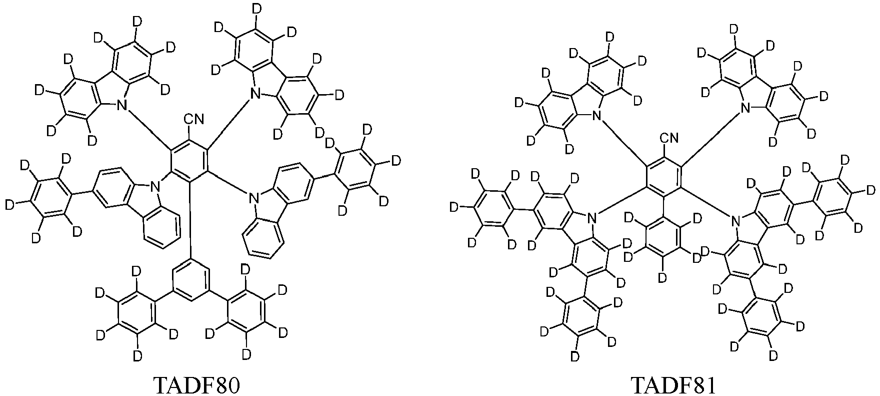

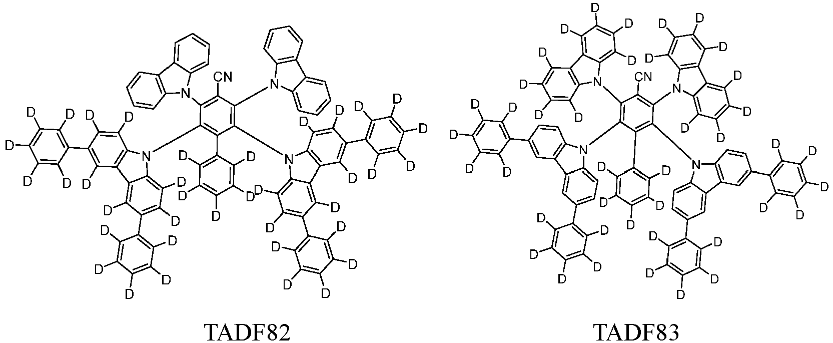

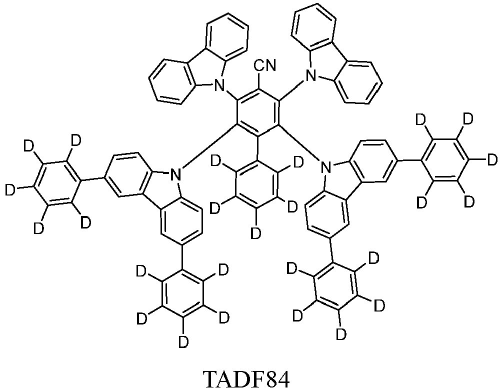

In the invention, a known delayed fluorescence material can be used in suitable combination with the compound represented by the formula (1), as well as the above. Further, an unknown delayed fluorescence material can also be used.

Examples of the delayed fluorescence material include compounds included in the general formulae described in WO2013/154064, paragraphs 0008 to 0048 and 0095 to 0133; WO2013/011954, paragraphs 0007 to 0047 and 0073 to 0085; WO2013/011955, paragraphs 0007 to 0033 and 0059 to 0066; WO2013/081088, paragraphs 0008 to 0071 and 0118 to 0133; JP 2013-256490 A, paragraphs 0009 to 0046 and 0093 to 0134; JP 2013-116975 A, paragraphs 0008 to 0020 and 0038 to 0040; WO2013/133359, paragraphs 0007 to 0032 and 0079 to 0084; WO2013/161437, paragraphs 0008 to 0054 and 0101 to 0121; JP 2014-9352 A, paragraphs 0007 to 0041 and 0060 to 0069; JP 2014-9224 A, paragraphs 0008 to 0048 and 0067 to 0076; JP 2017-119663 A, paragraphs 0013 to 0025; JP 2017-119664 A, paragraphs 0013 to 0026; JP 2017-222623 A, paragraphs 0012 to 0025; JP 2017-226838 A, paragraphs 0010 to 0050; JP 2018-100411 A, paragraphs 0012 to 0043; and WO2018/047853, paragraphs 0016 to 0044, and particularly, exemplary compounds therein capable of emitting delayed fluorescence. Further, light-emitting materials capable of emitting delayed fluorescence, as described in JP 2013-253121 A, WO2013/133359, WO2014/034535, WO2014/115743, WO2014/122895, WO2014/126200, WO2014/136758, WO2014/133121, WO2014/136860, WO2014/196585, WO2014/189122, WO2014/168101, WO2015/008580, WO2014/203840, WO2015/002213, WO2015/016200, WO2015/019725, WO2015/072470, WO2015/108049, WO2015/080182, WO2015/072537, WO2015/080183, JP 2015-129240 A, WO2015/129714, WO2015/129715, WO2015/133501, WO2015/136880, WO2015/137244, WO2015/137202, WO2015/137136, WO2015/146541, and WO2015/159541, can also be adopted. These patent publications described in this paragraph are hereby incorporated as a part of this description by reference.

It is preferable that the delayed fluorescence material that is used in the invention does not contain metal atoms. For example, as the delayed fluorescence material, a compound containing atoms selected from the group consisting of a carbon atom, a hydrogen atom, a nitrogen atom, an oxygen atom, and a sulfur atom can be selected. For example, as the delayed fluorescence material, a compound containing atoms selected from the group consisting of a carbon atom, a hydrogen atom, a nitrogen atom, and an oxygen atom can be selected. For example, as the delayed fluorescence material, a compound containing a carbon atom, a hydrogen atom, and a nitrogen atom can be selected.

In the present specification, an alkyl group, an alkenyl group, an aryl group, a heteroaryl group, and the like represent the following contents, unless otherwise noted.

The "alkyl group" may take any of linear, branched, and cyclic shapes. Further, two or more types among the linear portion, the cyclic portion, and the branched portion may be mixed. The number of carbon atoms of the alkyl group may be, for example, 1 or more, 2 or more, or 4 or more. Further, the number of carbon atoms may be 30 or less, 20 or less, 10 or less, 6 or less, or 4 or less. Specific examples of the alkyl group include a methyl group, an ethyl group, a n-propyl group, an isopropyl group, a n-butyl group, an isobutyl group, a tert-butyl group, a n-pentyl group, an isopentyl group, a n-hexyl group, an isohexyl group, a 2-ethyl hexyl group, a n-heptyl group, an isoheptyl group, a n-octyl group, an isooctyl group, a n-nonyl group, an isononyl group, a n-decanyl group, an isodecanyl group, a cyclopentyl group, a cyclohexyl group, and a cycloheptyl group. The alkyl group as a substituent may be further substituted with an aryl group. The alkyl group as a substituent may be further substituted with an aryl group. For alkyl portions of an "alkoxy group", an "alkylthio group", an "acyl group" and an "alkoxycarbonyl group", the description on the "alkyl group" mentioned herein can be referred to.

The "alkenyl group" may take any of linear, branched, and cyclic shapes. Further, two or more types among the linear portion, the cyclic portion, and the branched portion may be mixed. The number of carbon atoms of the alkenyl group may be, for example, 2 or more, or 4 or more. Further, the number of carbon atoms may be 30 or less, 20 or less, 10 or less, 6 or less, or 4 or less. Specific examples of the alkenyl group include an ethenyl group, a n-propenyl group, an isopropenyl group, a n-butenyl group, an isobutenyl group, a n-pentenyl group, an isopentenyl group, a n-hexenyl group, an isohexenyl group, and a 2-ethyl hexenyl group. The alkenyl group as a substituent may be further substituted with a substituent.

The "aryl group" and the "heteroaryl group" may be a monocycle, or may be a condensed ring in which two or more rings are condensed. In the case of a condensed ring, the number of rings that are condensed is preferably 2 to 6, and for example, can be selected from 2 to 4. Specific examples of the ring include a benzene ring, a pyridine ring, a pyrimidine ring, a triazine ring, a naphthalene ring, an anthracene ring, a phenanthrene ring, a triphenylene ring, a quinoline ring, a pyrazine ring, a quinoxaline ring, and a naphthyridine ring. Specific examples of the aryl group or the heteroaryl group include a phenyl group, a 1-naphthyl group, a 2-naphthyl group, a 1-anthracenyl group, a 2-anthracenyl group, a 9-anthracenyl group, a 2-pyridyl group, a 3-pyridyl group, and a 4-pyridyl group. The "arylene group" and the "heteroaryl group" may be those obtained by changing the valence in the descriptions for the aryl group and the heteroaryl group, from 1 to 2. For aryl portions of an "aryl oxy group", an "arylthio group" and an "aryl oxycarbonyl group", the description on the "aryl group" mentioned herein can be referred to. For heteroaryl portions of a "heteroaryl oxy group", a "heteroarylthio group" and a "heteroaryl oxycarbonyl group", the description on the "heteroaryl group" mentioned herein can be referred to.

### (Composition)

The composition of the invention contains the compound represented by the formula (1) and the delayed fluorescence material. In one aspect of the invention, the composition contains only one or more types of compounds represented by the formula (1), and one or more types of delayed fluorescence materials. In one aspect of the invention, the composition contains only one type of compound represented by the formula (1) and one type of delayed fluorescence material. In one aspect of the invention, the composition contains a third component other than the compound represented by the formula (1) and the delayed fluorescence material. The third component mentioned herein is not the compound represented by the formula (1), and is not the delayed fluorescence material. Only one type of third component may be contained, or two or more types thereof may be contained. The content of the third component in the composition may be selected from a range of 30% by weight or less, may be selected from a range of 10% by weight or less, may be selected from a range of 1% by weight or less, or may be selected from a range of 0.1% by weight or less. In one aspect of the invention, the third component does not emit light. In one aspect of the invention, the third component emits fluorescence. In a preferred aspect of the invention, the largest component of light emitted from the composition of the invention is fluorescence (including delayed fluorescence).

In the composition of the invention, the content of the compound represented by the formula (1) is larger than that of the delayed fluorescence material in the basis of weight. The content of the compound represented by the formula (1) may be selected from a range of 3 times by weight or more, may be selected from a range of 10 times by weight or more, may be selected from a range of 100 times by weight or more, or may be selected from a range of 1000 times by weight or more, and for example may be selected from a range of 10000 times by weight or less, with respect to the content of the delayed fluorescence material.

In the composition of the invention, it is preferable to select a delayed fluorescence material having excited singlet energy lower than the excited singlet energy of the compound represented by the formula (1). A difference in the excited singlet energy may be 0.1 eV or more, may be 0.3 eV or more, may be 0.5 eV or more, may be 2 eV or less, may be 1.5 eV or less, or may be 1.0 eV or less.

It is preferable that the composition of the invention does not contain a metal element. In one aspect of the invention, the composition of the invention consists of atoms selected from the group consisting of a carbon atom, a hydrogen atom, a nitrogen atom, an oxygen atom, a sulfur atom, a boron atom, and a halogen atom. In one aspect of the invention, the composition of the invention consists of atoms selected from the group consisting of a carbon atom, a hydrogen atom, a nitrogen atom, an oxygen atom, and a sulfur atom.

Further, in one aspect of the invention, the compound represented by the formula (1) is useful as a host material, for use together with the delayed fluorescence material and a fluorescent compound. Thus, in one aspect of the invention, the composition of the invention also contains the fluorescent compound as well as the compound represented by the formula (1) and the delayed fluorescence material.

It is preferable that the fluorescent compound has lowest excited singlet energy (E_{S1}) lower than that of the compound represented by the formula (1) and the delayed fluorescence material. The fluorescent compound receives the energy from the compound represented by the formula (1) and the delayed fluorescence material in the excited singlet state, and the delayed fluorescence material in the excited singlet state from the excited triplet state by inverse intersystem crossing to transition to the singlet excited state, and then, emits fluorescence when returning to the ground state. The fluorescent compound is not particularly limited insofar as the fluorescence can be emitted by receiving the energy from the compound represented by the formula (1) and the delayed fluorescence material, and the light to be emitted may be fluorescence, or may be delayed fluorescence. Among them, an illuminant that emits fluorescence when returning to the ground energy level from the lowest excited singlet energy level is preferable as an illuminant used as the fluorescent compound. Two or more types of fluorescent compounds may be used. For example, by using two or more types of fluorescent compounds with different luminescent colors together, it is possible to emit light with a desired color.

As the fluorescent compound, an anthracene derivative, a tetracene derivative, a naphthacene derivative, a pyrene derivative, a perylene derivative, a chrysene derivative, a rubrene derivative, a coumarin derivative, a pyrane derivative, a stilbene derivative, a fluorene derivative, an anthryl derivative, a pyrromethene derivative, a terphenyl derivative, a terphenylene derivative, a fluoranthene derivative, an amine derivative, a quinacridone derivative, an oxadiazole derivative, a malononitrile derivative, a pyrane derivative, a carbazole derivative, a julolidine derivative, a thiazole derivative, a derivative having metals (Al and Zn), a compound having a multiple resonance effect, such as a compound having a boron-containing polycyclic aromatic skeleton such as diazaboranaphthoanthracene, and the like can be used. Such exemplified skeletons may have a substituent, or may not have a substituent. Further, such exemplified skeletons may be combined with each other.

Specific examples of the fluorescent compound include the compounds given as the specific examples of the delayed fluorescence material. Here, the composition of the invention contains two or more types of delayed fluorescence materials, but a delayed fluorescence material with higher lowest excited singlet energy functions as an assist dopant, and a delayed fluorescence material with lower lowest excited singlet energy functions as a fluorescent compound that mainly emits light. The compound used as the fluorescent compound exhibits a PL emission quantum yield of preferably 60% or more, and more preferably 80% or more. Further, the compound used as the fluorescent compound exhibits the instantaneous fluorescence lifetime of preferably 50 ns or less, and more preferably 20 ns or less. The instantaneous fluorescence lifetime mentioned herein is the emission lifetime of a component that decays fastest among a plurality of exponential decay components observed when performing emission lifetime measurement with respect to a compound exhibiting thermal activation-type delayed fluorescence. Further, in the compound used as the third compound, it is preferable that a fluorescence emission rate from the lowest excited singlet (S1) to the ground state is higher than an intersystem crossing rate from S1 to the lowest excited triplet (T1). Regarding a method for calculating the rate constant of the compound, known documents relevant to a thermal activation-type delayed fluorescence material (H. Uoyama, et al., Nature 492, 234 (2012), K. Masui, et al., Org. Electron. 14, 2721, (2013), and the like) can be referred to.

Hereinafter, preferable compounds that can be used as the fluorescent compound used together with the delayed fluorescence material will be given, but the fluorescent compound used in the invention is not construed as limiting to such specific examples.

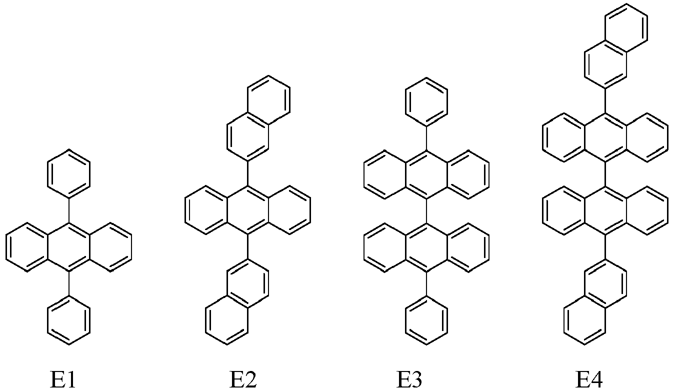

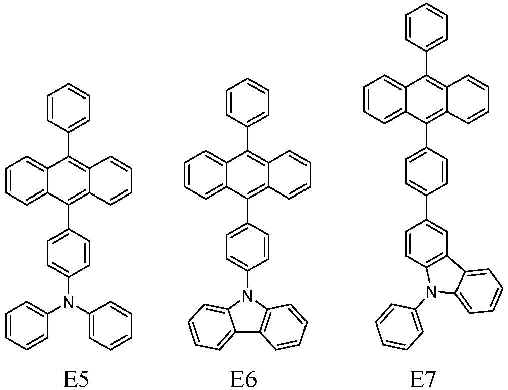

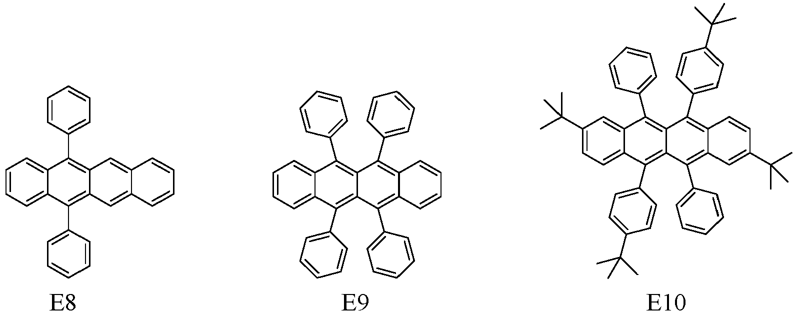

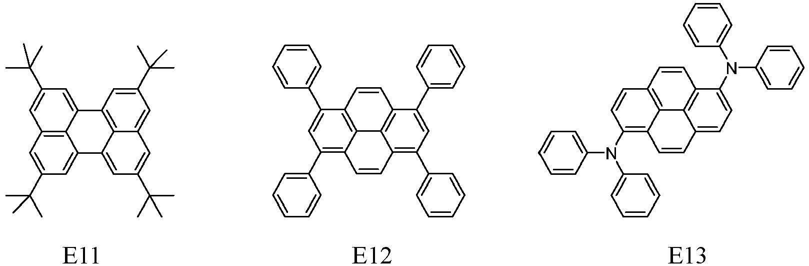

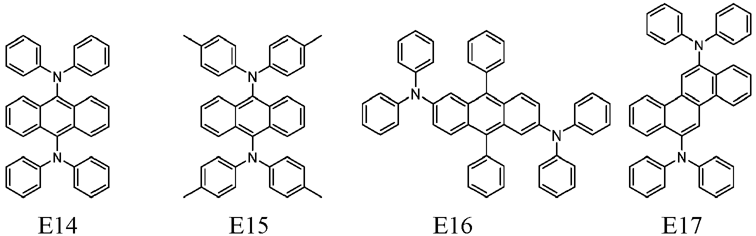

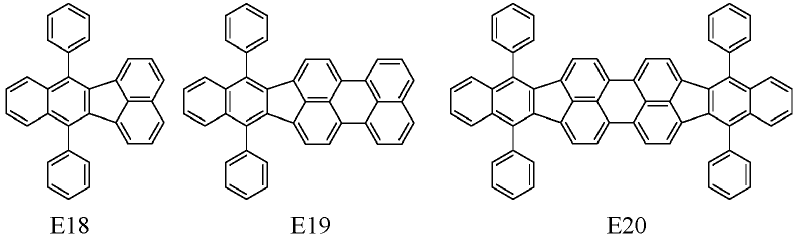

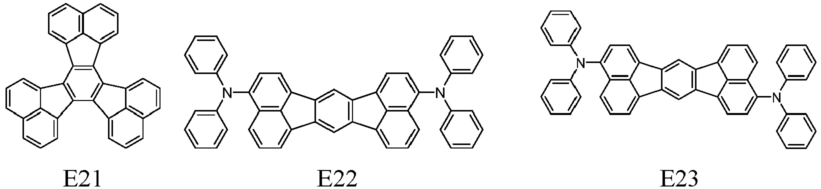

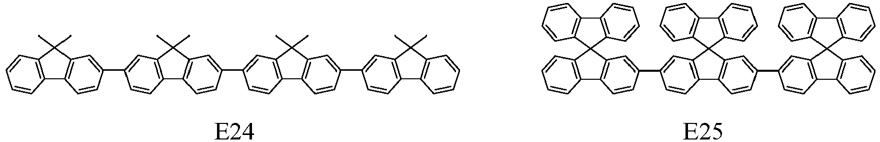

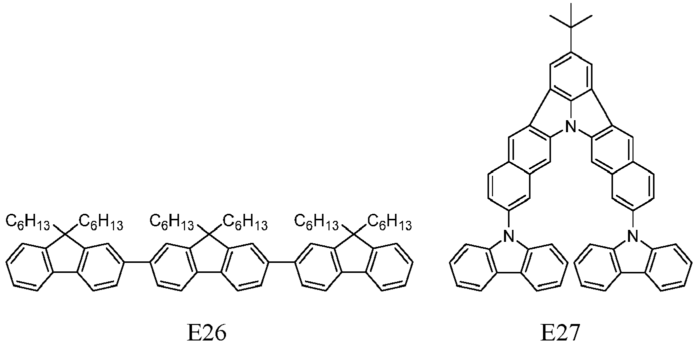

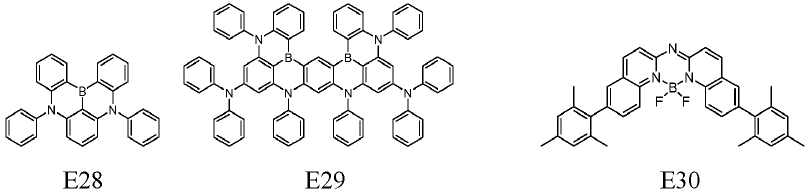

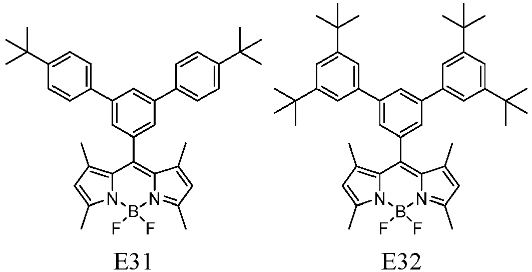

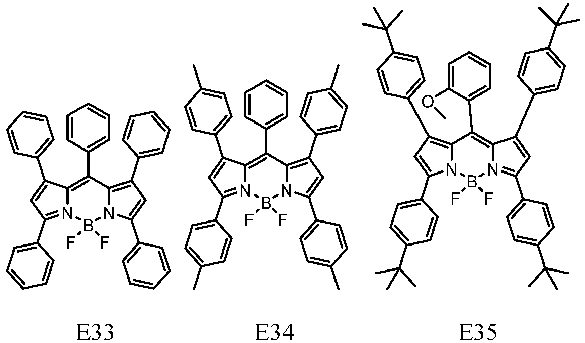

Further, a compound described in WO2015/022974, paragraphs 0220 to 0239, can also be particularly preferably adopted as the fluorescent compound of the invention.

Further, in one aspect of the invention, the compound represented by the formula (1) is used together with the other host material, and thus, can be used as the light-emitting layer (composition) containing a plurality of host materials. That is, in one aspect of the invention, the composition of the invention contains a plurality of host materials containing the compound represented by the formula (1). In the composition of the invention, a plurality of types of compounds represented by the formula (1) may be used, or the compound represented by the formula (1) and a host material that is not represented by the formula (1) may be used in combination.

Hereinafter, preferable compounds that can be used as a second host material used together with the compound represented by the formula (1) will be given, but the second host material that can be used in the invention is not construed as limiting to such specific examples.

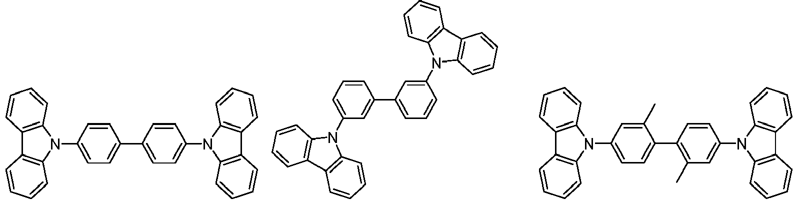

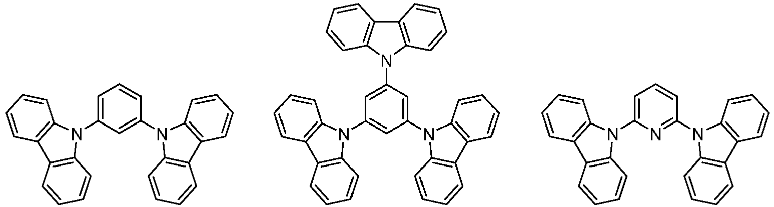

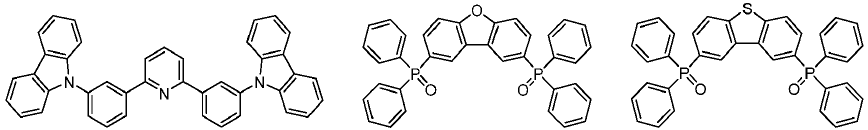

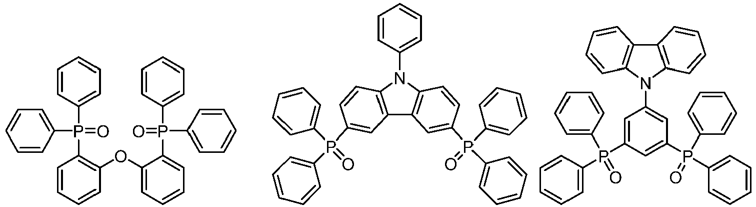

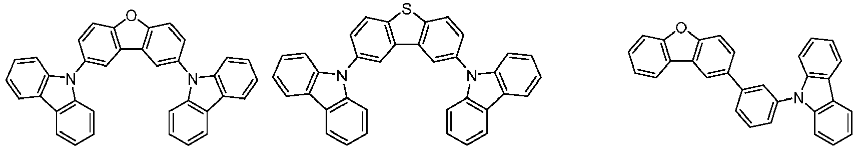

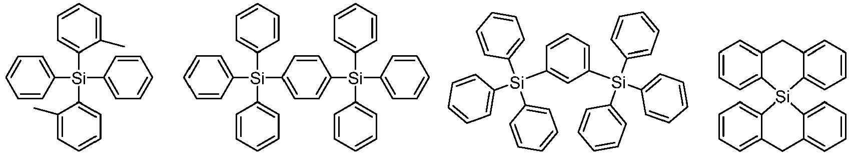

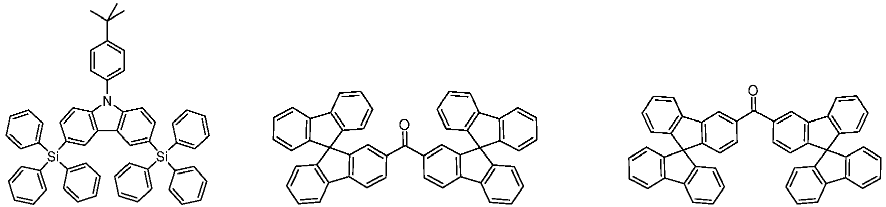

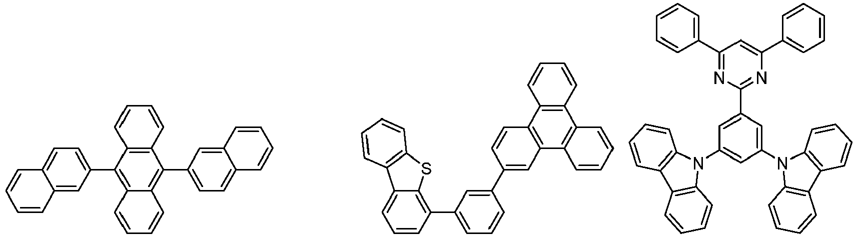

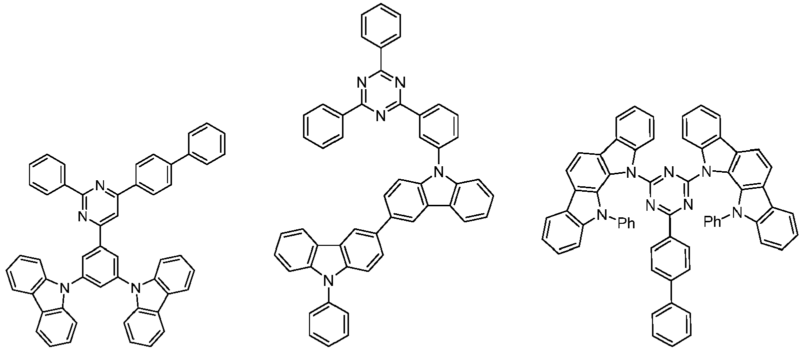

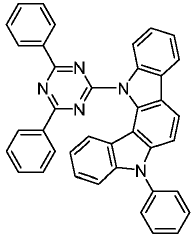

The form of the composition of the invention is not particularly limited. In a particularly preferred aspect of the invention, the composition of the invention is in the shape of a film. A film containing the composition of the invention may be formed in a wet process, or may be formed in a dry process.

In a wet process, a solution prepared by dissolving the composition of the invention is applied to a surface, and then the solvent is removed to form a light-emitting layer. The wet process includes a spin coating method, a slit coating method, an inkjet method (a spraying method), a gravure printing method, an offset printing method, and a flexographic printing method, which, however, are not limitative. In the wet process, an appropriate organic solvent capable of dissolving the composition of the invention is selected and used. In some embodiments, a substituent (for example, an alkyl group) capable of increasing the solubility in an organic solvent can be introduced into the compound to be contained in the composition of the invention.

A vacuum evaporation method is preferably employable as a dry process. In the case where a vacuum evaporation method is employed, each of compounds to constitute the composition of the invention can be co-evaporated from individual evaporation sources, or can be co-evaporated from a single evaporation source formed by mixing all of the compounds. In the case where a single evaporation source is used, a mixed powder prepared by mixing all of compound powders can be used, or a compression molded body prepared by compression-molding the mixed powder can be used, or a mixture prepared by heating and melting each of the compounds and cooling the resulting melt can be used. In some embodiments, by co-evaporation under the condition where the evaporation rate (weight reduction rate) of a plurality of compounds contained in a single evaporation source is the same or is nearly the same, a film having a compositional ratio corresponding to the compositional ratio of the plurality of compounds contained in the evaporation source can be formed. When the plurality of compounds are mixed in the same compositional ratio as the compositional ratio of the film to be formed to prepare an evaporation source, a film having a desired compositional ratio can be formed in a simplified manner. In some embodiments, the temperature at which each of the compounds to be co-evaporated has the same weight reduction ratio is specifically defined, and the temperature can be employed as the temperature of co-evaporation. When the film is formed by a vapor deposition method, the molecular weight of each of the compounds constituting the composition is preferably 1500 or less, more preferably 1200 or less, further preferably 1000 or less, and still further preferably 900 or less. The lower limit value of the molecular weight, for example, may be 450, may be 500, or may be 600.

### (Organic light-emitting device)

By forming the light-emitting layer containing the composition of the invention, it is possible to provide an excellent organic light-emitting device such as an organic photoluminescence device (organic PL device) or an organic electroluminescence device (organic EL device). The organic light-emitting device of the invention is a fluorescence emission device, and the largest component of the light emitted from the device is fluorescence (the fluorescence mentioned herein includes delayed fluorescence).

The thickness of the light-emitting layer, for example, can be 1 to 15 nm, can be 2 to 10 nm, or can be 3 to 7 nm.

The organic photoluminescence device has a structure in which at least the light-emitting layer is formed on a substrate. Further, the organic electroluminescence device has a structure in which at least an anode, a cathode, and an organic layer between the anode and the cathode are formed. The organic layer includes at least the light-emitting layer, may include only the light-emitting layer, or may include one or more organic layers as well as the light-emitting layer. Examples of such organic layers include a hole transport layer, a hole injection layer, an electron barrier layer, a hole barrier layer, an electron injection layer, an electron transport layer, and an exciton barrier layer. The hole transport layer may be a hole injection transport layer having a hole injection function, and the electron transport layer may be an electron injection transport layer having an electron injection function. A specific structural example of the organic electroluminescence device is illustrated in FIG. 1. In FIG. 1, 1 represents a substrate, 2 represents an anode, 3 represents a hole injection layer, 4 represents a hole transport layer, 5 represents a light-emitting layer, 6 represents an electron transport layer, and 7 represents a cathode.

When the organic light-emitting device of the invention is a multi-wavelength emission type organic light-emitting device, light emission with the shortest wavelength may include delayed fluorescence. Further, the light emission with the shortest wavelength may not include delayed fluorescence.

The organic light-emitting device using the composition of the invention is, when excited thermally or by an electronic means, able to emit light in a UV region, light of blue, green, yellow, or orange in a visible spectrum region, in a red region (e.g., 420 nm to 500 nm, 500 nm to 600 nm, or 600 nm to 700 nm) or in a near IR region. For example, the organic light-emitting device is able to emit light in a red or orange region (e.g., 620 to 780 nm). For example, the organic light-emitting device is able to emit light in an orange or yellow region (e.g., 570 to 620 nm). For example, the organic light-emitting device is able to emit light in a green region (e.g., 490 to 575 nm). For example, the organic light-emitting device is able to emit light in a blue region (e.g., 400 to 490 nm). For example, the organic light-emitting device is able to emit light in a UV spectrum region (e.g., 280 to 400 nm). For example, the organic light-emitting device is able to emit light in an IR spectrum region (e.g., 780 nm to 2 µm).

It is preferable that the largest component of light emitted from the organic light-emitting device using the composition of the invention is light emitted from the delayed fluorescence material contained in the composition of the invention. The light emitted from the compound represented by the formula (1) is preferably less than 10% of the light emitted from the organic light-emitting device, for example, may be less than 1%, less than 0.1%, less than 0.01%, or a detection limit or less. The light emitted from the delayed fluorescence material may be, for example, greater than 50%, greater than 90%, or greater than 99% of the light emitted from the organic light-emitting device. In the case where a layer containing the composition of the invention (the light-emitting layer) contains the fluorescence material as the third component, the largest component of the light emitted from the organic light-emitting device may be light emitted from the fluorescence material. In such a case, the light emitted from the light-emitting material may be, for example, greater than 50%, greater than 90%, or greater than 99% of the light emitted from the organic light-emitting device.

In the following, the constituent members and the other layers than a light-emitting layer of the organic electroluminescent device are described.

### Substrate:

In some embodiments, the organic electroluminescent device of the invention is supported by a substrate, wherein the substrate is not particularly limited and may be any of those that have been commonly used in an organic electroluminescent device, for example those formed of glass, transparent plastics, quartz, and silicon.

### Anode:

In some embodiments, the anode of the organic electroluminescent device is made of a metal, an alloy, an electroconductive compound, or a combination thereof. In some embodiments, the metal, alloy, or electroconductive compound has a large work function (4 eV or more). In some embodiments, the metal is Au. In some embodiments, the electroconductive transparent material is selected from CuI, indium tin oxide (ITO), SnO₂, and ZnO. In some embodiments, an amorphous material capable of forming a transparent electroconductive film, such as IDIXO (In₂O₃-ZnO), is used. In some embodiments, the anode is a thin film. In some embodiments, the thin film is made by vapor deposition or sputtering. In some embodiments, the film is patterned by a photolithography method. In some embodiments, when the pattern may not require high accuracy (for example, approximately 100 µm or more), the pattern may be formed with a mask having a desired shape on vapor deposition or sputtering of the electrode material. In some embodiments, when a material can be applied as a coating material, such as an organic electroconductive compound, a wet film forming method, such as a printing method and a coating method is used. In some embodiments, when the emitted light goes through the anode, the anode has a transmittance of more than 10%, and the anode has a sheet resistance of several hundred Ohm per square or less. In some embodiments, the thickness of the anode is from 10 to 1,000 nm. In some embodiments, the thickness of the anode is from 10 to 200 nm. In some embodiments, the thickness of the anode varies depending on the material used.

### Cathode:

In some embodiments, the cathode is made of an electrode material such as a metal having a small work function (4 eV or less) (referred to as an electron injection metal), an alloy, an electroconductive compound, or a combination thereof. In some embodiments, the electrode material is selected from sodium, a sodium-potassium alloy, magnesium, lithium, a magnesium-cupper mixture, a magnesium-silver mixture, a magnesium-aluminum mixture, a magnesium-indium mixture, an aluminum-aluminum oxide (Al₂O₃) mixture, indium, a lithium-aluminum mixture, and a rare earth metal. In some embodiments, a mixture of an electron injection metal and a second metal that is a stable metal having a larger work function than the electron injection metal is used. In some embodiments, the mixture is selected from a magnesium-silver mixture, a magnesium-aluminum mixture, a magnesium-indium mixture, an aluminum-aluminum oxide (Al₂O₃) mixture, a lithium-aluminum mixture, and aluminum. In some embodiments, the mixture increases the electron injection property and the durability against oxidation. In some embodiments, the cathode is produced by forming the electrode material into a thin film by vapor deposition or sputtering. In some embodiments, the cathode has a sheet resistance of several hundred Ohm per square or less. In some embodiments, the thickness of the cathode ranges from 10 nm to 5 µm. In some embodiments, the thickness of the cathode ranges from 50 to 200 nm. In some embodiments, for transmitting the emitted light, any one of the anode and the cathode of the organic electroluminescent device is transparent or translucent. In some embodiments, the transparent or translucent electroluminescent devices enhance the light emission luminance.

In some embodiments, the cathode is formed with an electroconductive transparent material, as described for the anode, to form a transparent or translucent cathode. In some embodiments, a device comprises an anode and a cathode, both being transparent or translucent.

### Injection Layer:

An injection layer is a layer between the electrode and the organic layer. In some embodiments, the injection layer decreases the driving voltage and enhances the light emission luminance. In some embodiments, the injection layer includes a hole injection layer and an electron injection layer. The injection layer can be positioned between the anode and the light-emitting layer or the hole transport layer, and between the cathode and the light-emitting layer or the electron transport layer. In some embodiments, an injection layer is present. In some embodiments, no injection layer is present.

Preferred compound examples for use as a hole injection material are shown below.

### MoO₃,

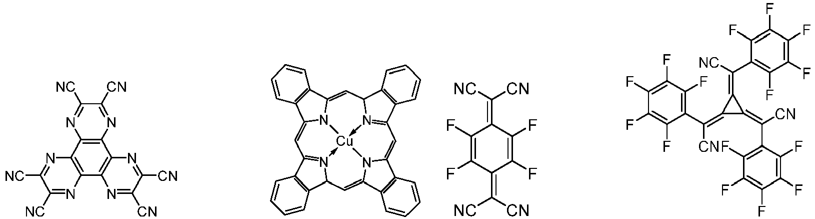

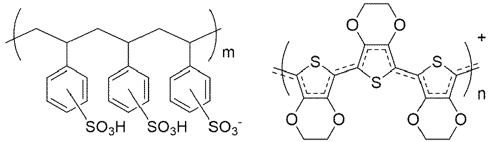

Next, preferred compound examples for use as an electron injection material are shown below. LiF, CsF,

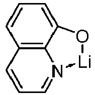

### Barrier Layer:

A barrier layer is a layer capable of inhibiting charges (electrons or holes) and/or excitons present in the light-emitting layer from being diffused outside the light-emitting layer. In some embodiments, an electron barrier layer is between the light-emitting layer and the hole transport layer, and inhibits electrons from passing through the light-emitting layer toward the hole transport layer. In some embodiments, a hole barrier layer is between the light-emitting layer and the electron transport layer, and inhibits holes from passing through the light-emitting layer toward the electron transport layer. In some embodiments, the barrier layer inhibits excitons from being diffused outside the light-emitting layer. In some embodiments, the electron barrier layer and the hole barrier layer are exciton barrier layers. As used herein, the term "electron barrier layer" or "exciton barrier layer" includes a layer that has the functions of both electron barrier layer and of an exciton barrier layer.

### Hole Barrier Layer:

A hole barrier layer acts as an electron transport layer. In some embodiments, the hole barrier layer inhibits holes from reaching the electron transport layer while transporting electrons. In some embodiments, the hole barrier layer enhances the recombination probability of electrons and holes in the light-emitting layer. The material for the hole barrier layer may be the same materials as the ones described for the electron transport layer.

Preferred compound examples for use for the hole barrier layer are shown below.

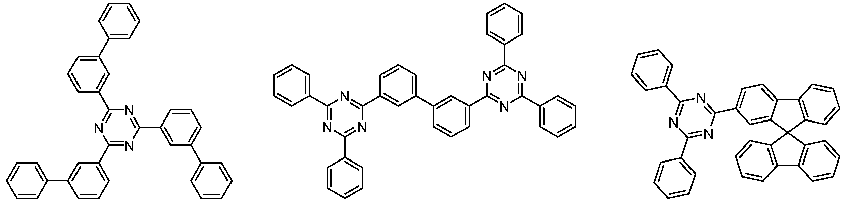

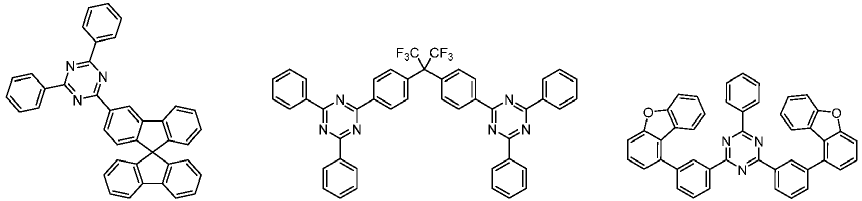

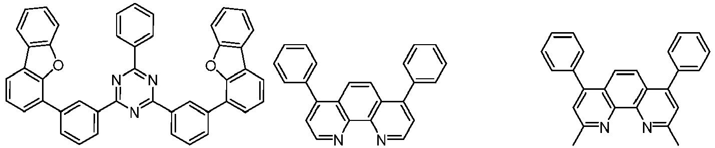

### Electron Barrier Layer:

An electron barrier layer transports holes. In some embodiments, the electron barrier layer inhibits electrons from reaching the hole transport layer while transporting holes. In some embodiments, the electron barrier layer enhances the recombination probability of electrons and holes in the light-emitting layer. The materials for use for the electron barrier layer may be the same materials as those mentioned herein above for the hole transport layer.

Preferred compound examples for use as the electron barrier material are shown below.

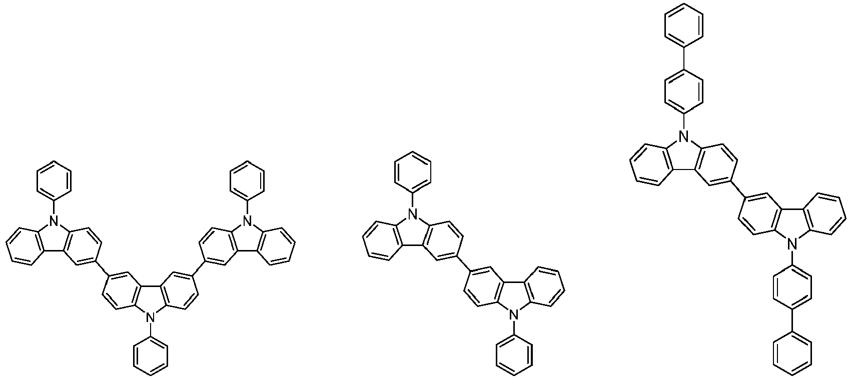

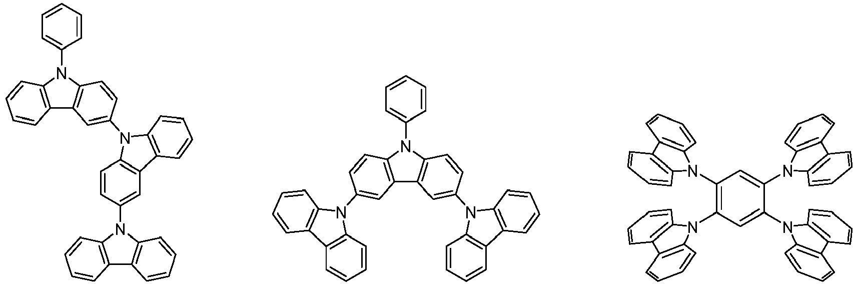

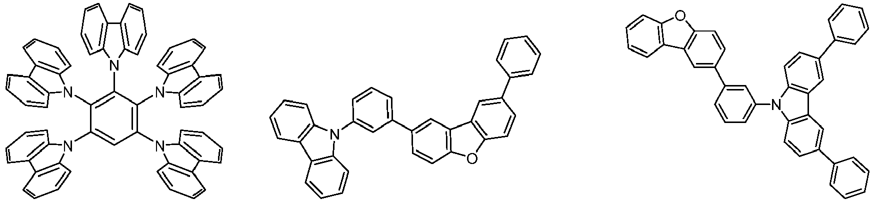

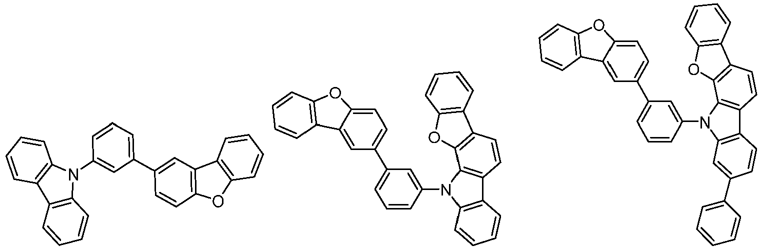

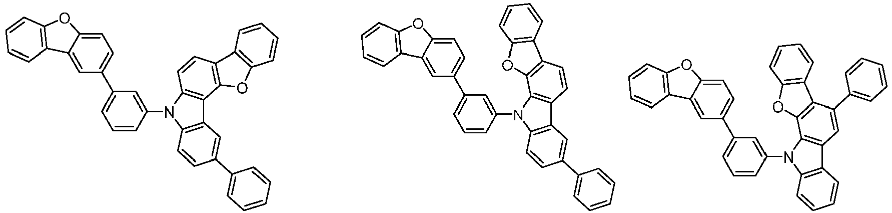

### Exciton Barrier Layer:

An exciton barrier layer inhibits excitons generated through recombination of holes and electrons in the light-emitting layer from being diffused to a charge transport layer. In some embodiments, the exciton barrier layer enables effective confinement of excitons in the light-emitting layer. In some embodiments, the light emission efficiency of a device is enhanced. In some embodiments, the exciton barrier layer is adjacent to the light-emitting layer on any of the side of the anode and the side of the cathode, and on both the sides. In some embodiments, when the exciton barrier layer is on the side of the anode, the layer can be between the hole transport layer and the light-emitting layer and adjacent to the light-emitting layer. In some embodiments, when the exciton barrier layer is on the side of the cathode, the layer can be between the light-emitting layer and the cathode and adjacent to the light-emitting layer. In some embodiments, a hole injection layer, an electron barrier layer, or a similar layer is between the anode and the exciton barrier layer that is adjacent to the light-emitting layer on the side of the anode. In some embodiments, a hole injection layer, an electron barrier layer, a hole barrier layer, or a similar layer is between the cathode and the exciton barrier layer that is adjacent to the light-emitting layer on the side of the cathode. In some embodiments, the exciton barrier layer comprises excited singlet energy and excited triplet energy, at least one of which is higher than the excited singlet energy and the excited triplet energy of the light-emitting material, respectively.

### hole transport layer :

The hole transport layer comprises a hole transport material. In some embodiments, the hole transport layer is a single layer. In some embodiments, the hole transport layer comprises a plurality of layers.

In some embodiments, the hole transport material has one of injection or transport property of holes and barrier property of electrons. In some embodiments, the hole transport material is an organic material. In some embodiments, the hole transport material is an inorganic material. Examples of known hole transport materials that may be used in the invention include but are not limited to a triazole derivative, an oxadiazole inducer, an imidazole derivative, a carbazole derivative, an indolocarbazole derivative, a polyaryl alkane inducer, a pyrazoline derivative, a pyrazolone derivative, a phenylene diamine derivative, an arylamine derivative, an amino-substituted chalcone derivative, an oxazole derivative, a styryl anthracene inducer, a fluorenone derivative, a hydrazone derivative, a stilbene derivative, a silazane derivative, an aniline copolymer and an electroconductive polymer oligomer, particularly a thiophene oligomer, or a combination thereof. In some embodiments, the hole transport material is selected from a porphyrin compound, an aromatic tertiary amine compound, and a styryl amine compound. In some embodiments, the hole transport material is an aromatic tertiary amine compound. Preferred compound examples for use as the hole transport material are shown below.

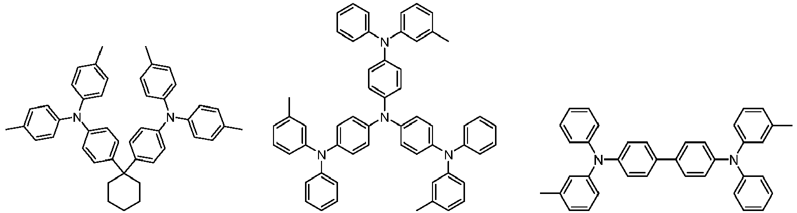

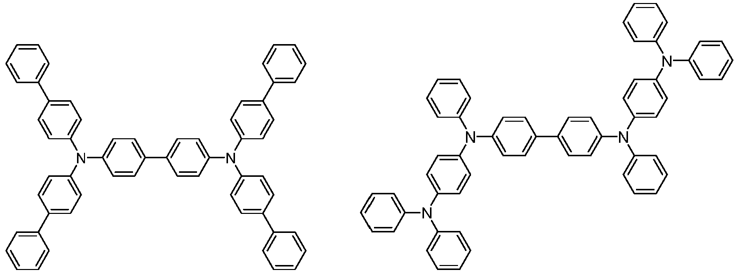

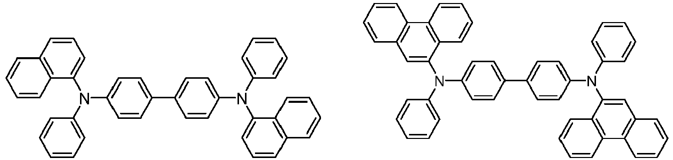

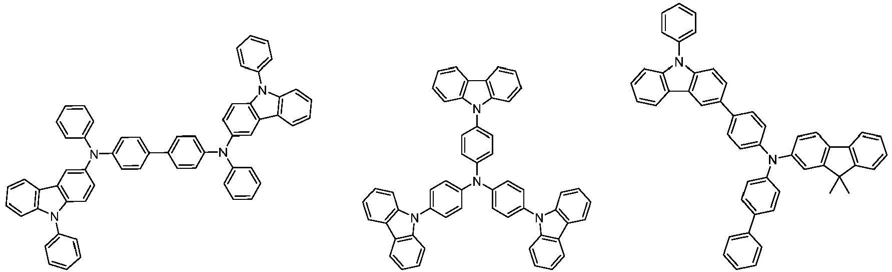

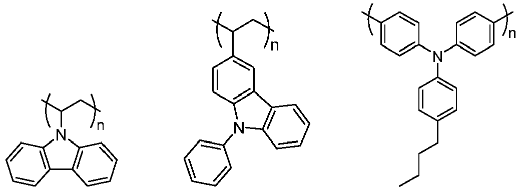

### Electron Transport Layer:

The electron transport layer comprises an electron transport material. In some embodiments, the electron transport layer is a single layer. In some embodiments, the electron transport layer comprises a plurality of layers.

In some embodiments, the electron transport material needs only to have a function of transporting electrons, which are injected from the cathode, to the light-emitting layer. In some embodiments, the electron transport material also functions as a hole barrier material. Examples of the electron transport layer that may be used here the invention include but are not limited to a nitro-substituted fluorene derivative, a diphenyl quinone derivative, a thiopyran dioxide derivative, carbodiimide, a fluorenylidene methane derivative, anthraquinodimethane, an anthrone derivatives, an oxadiazole derivative, an azole derivative, an azine derivative, or a combination thereof, or a polymer thereof. In some embodiments, the electron transport material is a thiadiazole inducer, or a quinoxaline derivative. In some embodiments, the electron transport material is a polymer material. Preferred compound examples for use as the electron transport material are shown below.

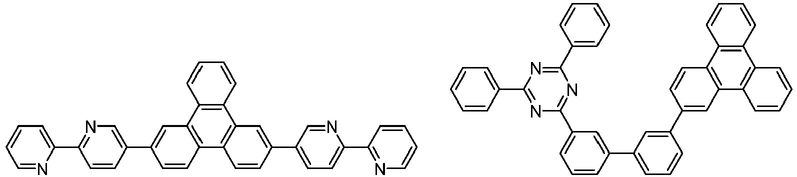

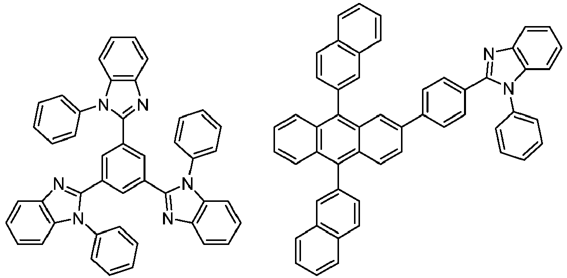

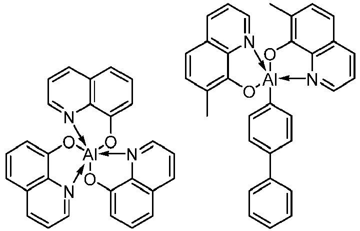

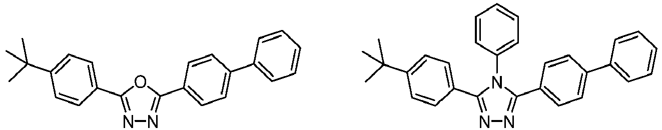

Preferred examples of compounds usable as materials that can be added to each organic layer are shown below. For example, the addition of a compound as a stabilizing material may be taken into consideration.

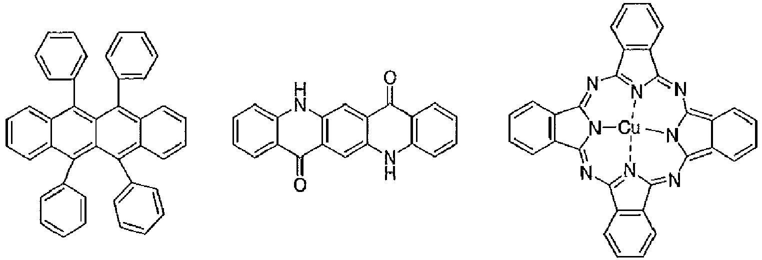

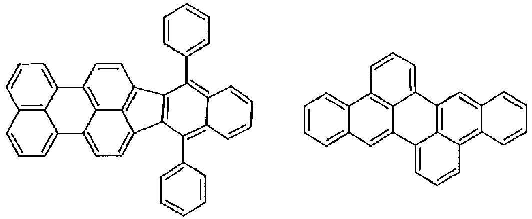

Preferred materials for use in an organic electroluminescent device are specifically shown. However, the materials usable in the invention should not be limitatively interpreted by the following exemplary compounds. Compounds that are exemplified as materials having a specific function can also be used as materials having any other function.

### Device:

In some embodiments, the light-emitting layers are incorporated into a device. For example, the device includes, but is not limited to an OLED bulb, an OLED lamp, a television screen, a computer monitor, a mobile phone, and a tablet.

In some embodiments, an electronic device comprises an OLED comprising an anode, a cathode, and at least one organic layer comprising a light-emitting layer between the anode and the cathode.

In some embodiments, compositions described herein may be incorporated into various light-sensitive or light-activated devices, such as OLEDs or photovoltaic devices. In some embodiments, the composition may be useful in facilitating charge transfer or energy transfer within a device and/or as a hole transport material. The device may be, for example, an organic light-emitting diode (OLED), an organic integrated circuit (OIC), an organic field-effect transistor (O-FET), an organic thin-film transistor (O-TFT), an organic light-emitting transistor (O-LET), an organic solar cell (O-SC), an organic optical detector, an organic photoreceptor, an organic field-quench device (O-FQD), a light-emitting electrochemical cell (LEC) or an organic laser diode (O-laser).

### Bulbs or Lamps:

In some embodiments, an electronic device comprises an OLED comprising an anode, a cathode, and at least one organic layer comprising a light-emitting layer between the anode and the cathode.

In some embodiments, a device comprises OLEDs that differ in color. In some embodiments, a device comprises an array comprising a combination of OLEDs. In some embodiments, the combination of OLEDs is a combination of three colors (e.g., RGB). In some embodiments, the combination of OLEDs is a combination of colors that are not red, green, or blue (e.g., orange and yellow green). In some embodiments, the combination of OLEDs is a combination of two, four, or more colors.

In some embodiments, a device is an OLED light comprising,
a circuit board having a first side with a mounting surface and an opposing second side, and defining at least one aperture;
at least one OLED on the mounting surface, the at least one OLED configured to emanate light, comprising an anode, a cathode, and at least one organic layer comprising a light-emitting layer between the anode and the cathode;
a housing for the circuit board; and
at least one connector arranged at an end of the housing, the housing and the connector defining a package adapted for installation in a light fixture.

In some embodiments, the OLED light comprises a plurality of OLEDs mounted on a circuit board such that light emanates in a plurality of directions. In some embodiments, a portion of the light emanated in a first direction is deflected to emanate in a second direction. In some embodiments, a reflector is used to deflect the light emanated in a first direction.

### Displays or Screens:

In some embodiments, the light-emitting layer in the invention can be used in a screen or a display. In some embodiments, the compounds in the invention are deposited onto a substrate using a process including, but not limited to, vacuum evaporation, deposition, vapor deposition, or chemical vapor deposition (CVD). In some embodiments, the substrate is a photoplate structure useful in a two-sided etch that provides a unique aspect ratio pixel. The screen (which may also be referred to as a mask) is used in a process in the manufacturing of OLED displays. The corresponding artwork pattern design facilitates a very steep and narrow tie-bar between the pixels in the vertical direction and a large, sweeping bevel opening in the horizontal direction. This allows the close patterning of pixels needed for high definition displays while optimizing the chemical deposition onto a TFT backplane.

The internal patterning of the pixel allows the construction of a 3-dimensional pixel opening with varying aspect ratios in the horizontal and vertical directions. Additionally, the use of imaged "stripes" or halftone circles within the pixel area inhibits etching in specific areas until these specific patterns are undercut and fall off the substrate. At that point, the entire pixel area is subjected to a similar etch rate but the depths are varying depending on the halftone pattern. Varying the size and spacing of the halftone pattern allows etching to be inhibited at different rates within the pixel allowing for a localized deeper etch needed to create steep vertical bevels.

A preferred material for the deposition mask is invar. Invar is a metal alloy that is cold rolled into long thin sheet in a steel mill. Invar cannot be electrodeposited onto a rotating mandrel as the nickel mask. A preferred and more cost feasible method for forming the open areas in the mask used for deposition is through a wet chemical etching.

In some embodiments, a screen or display pattern is a pixel matrix on a substrate. In some embodiments, a screen or display pattern is fabricated using lithography (e.g., photolithography and e-beam lithography). In some embodiments, a screen or display pattern is fabricated using a wet chemical etch. In further embodiments, a screen or display pattern is fabricated using plasma etching.

### Methods of Manufacturing Devices:

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light-emitting layer, a counter electrode, and an encapsulation layer, and then is cut from the mother panel.

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light-emitting layer, a counter electrode, and an encapsulation layer, and then is cut from the mother panel.

In another aspect of the invention, provided is a method of manufacturing an organic light-emitting diode (OLED) display, the method comprising:
forming a barrier layer on a base substrate of a mother panel;
forming a plurality of display units in units of cell panels on the barrier layer;
forming an encapsulation layer on each of the display units of the cell panels; and
applying an organic film to an interface portion between the cell panels.

In some embodiments, the barrier layer is an inorganic film formed of, for example, SiNx, and an edge portion of the barrier layer is covered with an organic film formed of polyimide or acryl. In some embodiments, the organic film helps the mother panel to be softly cut in units of the cell panel.

In some embodiments, the thin film transistor (TFT) layer includes a light-emitting layer, a gate electrode, and a source/drain electrode. Each of the plurality of display units may include a thin film transistor (TFT) layer, a planarization film formed on the TFT layer, and a light-emitting unit formed on the planarization film, wherein the organic film applied to the interface portion is formed of a same material as a material of the planarization film and is formed at a same time as the planarization film is formed. In some embodiments, the light-emitting unit is connected to the TFT layer with a passivation layer and a planarization film therebetween and an encapsulation layer that covers and protects the light-emitting unit. In some embodiments of the method of manufacturing, the organic film connects to neither the display units nor the encapsulation layer.

Each of the organic film and the planarization film may include any one of polyimide and acryl. In some embodiments, the barrier layer may be an inorganic film. In some embodiments, the base substrate may be formed of polyimide. The method may further include, before the forming of the barrier layer on one surface of the base substrate formed of polyimide, attaching a carrier substrate formed of a glass material to another surface of the base substrate, and before the cutting along the interface portion, separating the carrier substrate from the base substrate. In some embodiments, the OLED display is a flexible display.

In some embodiments, the passivation layer is an organic film disposed on the TFT layer to cover the TFT layer. In some embodiments, the planarization film is an organic film formed on the passivation layer. In some embodiments, the planarization film is formed of polyimide or acryl, like the organic film formed on the edge portion of the barrier layer. In some embodiments, the planarization film and the organic film are simultaneously formed when the OLED display is manufactured. In some embodiments, the organic film may be formed on the edge portion of the barrier layer such that a portion of the organic film directly contacts the base substrate and a remaining portion of the organic film contacts the barrier layer while surrounding the edge portion of the barrier layer.

In some embodiments, the light-emitting layer includes a pixel electrode, a counter electrode, and an organic light-emitting layer disposed between the pixel electrode and the counter electrode. In some embodiments, the pixel electrode is connected to the source/drain electrode of the TFT layer.

In some embodiments, when a voltage is applied to the pixel electrode through the TFT layer, an appropriate voltage is formed between the pixel electrode and the counter electrode, and thus the organic light-emitting layer emits light, thereby forming an image. Hereinafter, an image forming unit including the TFT layer and the light-emitting unit is referred to as a display unit.

In some embodiments, the encapsulation layer that covers the display unit and prevents penetration of external moisture may be formed to have a thin film encapsulation structure in which an organic film and an inorganic film are alternately stacked. In some embodiments, the encapsulation layer has a thin film encapsulation structure in which a plurality of thin films are stacked. In some embodiments, the organic film applied to the interface portion is spaced apart from each of the plurality of display units. In some embodiments, the organic film is formed such that a portion of the organic film directly contacts the base substrate and a remaining portion of the organic film contacts the barrier layer while surrounding an edge portion of the barrier layer.

In one embodiment, the OLED display is flexible and uses a soft base substrate formed of polyimide. In some embodiments, the base substrate is formed on a carrier substrate formed of a glass material, and then the carrier substrate is separated.

In some embodiments, the barrier layer is formed on a surface of the base substrate opposite to the carrier substrate. In one embodiment, the barrier layer is patterned according to a size of each of the cell panels. For example, while the base substrate is formed over the entire surface of a mother panel, the barrier layer is formed according to a size of each of the cell panels, and thus a groove is formed at an interface portion between the barrier layers of the cell panels. Each of the cell panels can be cut along the groove.

In some embodiments, the method of manufacture further comprises cutting along the interface portion, wherein a groove is formed in the barrier layer, wherein at least a portion of the organic film is formed in the groove, and wherein the groove does not penetrate into the base substrate. In some embodiments, the TFT layer of each of the cell panels is formed, and the passivation layer which is an inorganic film and the planarization film which is an organic film are disposed on the TFT layer to cover the TFT layer. At the same time as the planarization film formed of, for example, polyimide or acryl is formed, the groove at the interface portion is covered with the organic film formed of, for example, polyimide or acryl. This is to prevent cracks from occurring by allowing the organic film to absorb an impact generated when each of the cell panels is cut along the groove at the interface portion. That is, when the entire barrier layer is entirely exposed without the organic film, an impact generated when each of the cell panels is cut along the groove at the interface portion is transferred to the barrier layer, thereby increasing the risk of cracks. However, in one embodiment, since the groove at the interface portion between the barrier layers is covered with the organic film and the organic film absorbs an impact that would otherwise be transferred to the barrier layer, each of the cell panels may be softly cut and cracks may be prevented from occurring in the barrier layer. In one embodiment, the organic film covering the groove at the interface portion and the planarization film are spaced apart from each other. For example, when the organic film and the planarization film are connected to each other as one layer, since external moisture may penetrate into the display unit through the planarization film and a portion where the organic film remains, the organic film and the planarization film are spaced apart from each other such that the organic film is spaced apart from the display unit.

In some embodiments, the display unit is formed by forming the light-emitting unit, and the encapsulation layer is disposed on the display unit to cover the display unit. As such, once the mother panel is completely manufactured, the carrier substrate that supports the base substrate is separated from the base substrate. In some embodiments, when a laser beam is emitted toward the carrier substrate, the carrier substrate is separated from the base substrate due to a difference in a thermal expansion coefficient between the carrier substrate and the base substrate.

In some embodiments, the mother panel is cut in units of the cell panels. In some embodiments, the mother panel is cut along an interface portion between the cell panels by using a cutter. In some embodiments, since the groove at the interface portion along which the mother panel is cut is covered with the organic film, the organic film absorbs an impact during the cutting. In some embodiments, cracks may be prevented from occurring in the barrier layer during the cutting.

In some embodiments, the methods reduce a defect rate of a product and stabilize its quality.

Another aspect is an OLED display including: a barrier layer that is formed on a base substrate; a display unit that is formed on the barrier layer; an encapsulation layer that is formed on the display unit; and an organic film that is applied to an edge portion of the barrier layer.

### Examples

The features of the invention will be described more specifically with reference to Synthesis Examples and Examples given below. The materials, processes, procedures and the like shown below may be appropriately modified unless they deviate from the substance of the invention. Accordingly, the scope of the invention is not construed as being limited to the specific examples shown below. Herein under the light emission characteristics were evaluated using a source meter (available from Keithley Instruments Corporation: 2400 series), a semiconductor parameter analyzer (available from Agilent Corporation, E5273A), an optical power meter device (available from Newport Corporation, 1930C), an optical spectroscope (available from Ocean Optics Corporation, USB2000), a spectroradiometer (available from Topcon Corporation, SR-3), and a streak camera (available from Hamamatsu Photonics K.K., Model C4334). Further, HOMO energy and LUMO energy were measured with a photo-electron spectroscopy in air (available from RKI INSTRUMENTS, INC., AC-3 or the like).

In the following Synthesis Examples, compounds to be included in the formula (1) were synthesized.

### (Synthesis Example 1) Synthesis of Compound 2

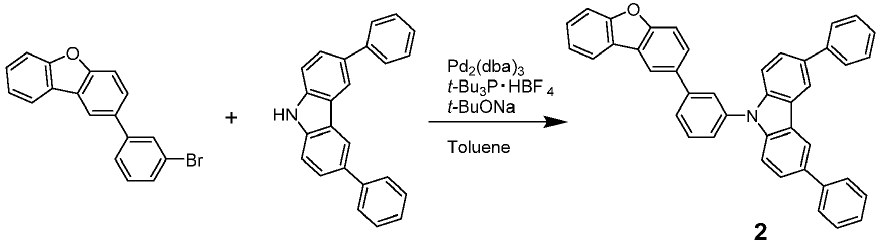

Under a nitrogen stream, 2-(3-bromophenyl) dibenzofuran (20 g, 61.9 mmol), 3,6-diphenyl carbazole (19.8 g, 61.9 mmol), tris(dibenzylidene acetone) dipalladium (0) (5.67 g, 6.19 mmol), tri-tert-butyl phosphonium tetrafluoroborate (3.59 g, 12.4 mmol) and sodium tert-butoxide (11.9 g, 123.8 mmol) were added to 400 ml of toluene, followed by refluxing for 24 h. The reaction solution was cooled to room temperature, and chloroform was added thereto. The resultant organic layer was washed with water two times and dried with magnesium sulfate to remove the solvent. The resultant solid was purified with silica gel column chromatography (a developing solvent: chloroform/n-hexane=3:7) and further recrystallized (toluene/methanol) to obtain a white solid (20 g, 58%).

¹H NMR (400 MHz, CDCl₃, δ): 8.43 (s, 2H), 8.22 (s, 1H), 7.99 (d, J = 8 Hz, 1H), 7.94 (s, 1H), 7.84-7.57 (m, 14H), 7.49 (m, 5H), 7.37 (m, 3H).

MS (ASAP): 562.32 (M+H⁺). Calcd for C₄₂H₂₇NO: 561.21.

### (Synthesis Example 2) Synthesis of Compound 10

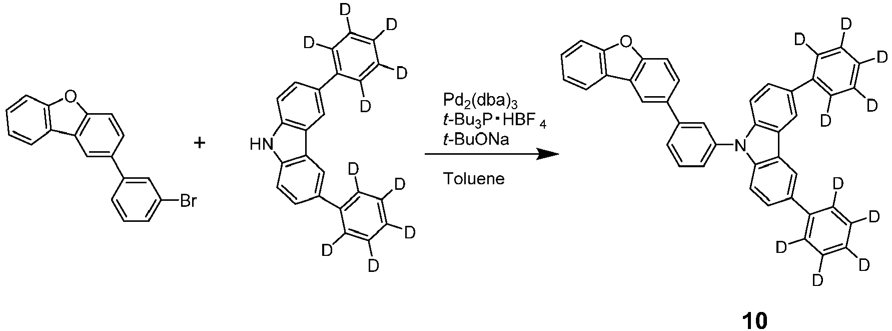

Under a nitrogen stream, 2-(3-bromophenyl) dibenzofuran (1.96 g, 6.07 mmol), 3,6-di(phenyl-2,3,4,5,6-d5)-9H-carbazole (2 g, 6.07 mmol), tris(dibenzylidene acetone) dipalladium (0) (0.56 g, 0.61 mmol), tri-tert-butyl phosphonium tetrafluoroborate (0.35 g, 1.21 mmol) and sodium tert-butoxide (1.17 g, 12.1 mmol) were added to 40 ml of toluene, followed by refluxing for 24 h. The reaction solution was cooled to room temperature, and chloroform was added thereto. The resultant organic layer was washed with water two times and dried with magnesium sulfate to remove the solvent. The resultant solid was purified with silica gel column chromatography (a developing solvent: chloroform/n-hexane=3:7) and further recrystallized (toluene/methanol) to obtain a white solid (2.55 g, 73%).

¹H NMR (400 MHz, CDCl₃, δ): 8.43 (s, 2H), 8.22 (s, 1H), 7.99 (d, J = 8 Hz, 1H), 7.94 (s, 1H), 7.84-7.56 (m, 10H), 7.49 (t, J = 8 Hz, 1H), 7.37 (t, J = 8 Hz, 1H).

MS (ASAP): 572.40 (M+H⁺). Calcd for C₄₂H₁₇D₁₀NO: 571.27.

### (Synthesis Example 3) Synthesis of Compound 30

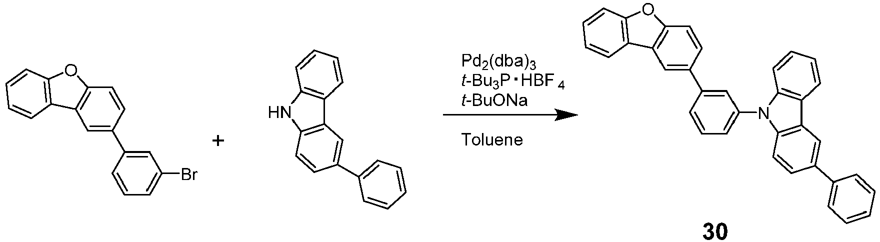

Under a nitrogen stream, 2-(3-bromophenyl) dibenzofuran (2 g, 6.19 mmol), 3-phenyl carbazole (1.51 g, 6.19 mmol), tris(dibenzylidene acetone) dipalladium (0) (0.57 g, 0.62 mmol), tri-tert-butyl phosphonium tetrafluoroborate (0.36 g, 1.24 mmol) and sodium tert-butoxide (1.19 g, 12.4 mmol) were added to 50 ml of toluene, followed by refluxing for 24 h. The reaction solution was cooled to room temperature, and chloroform was added thereto. The resultant organic layer was washed with water two times and dried with magnesium sulfate to remove the solvent. The resultant solid was purified with silica gel column chromatography (a developing solvent: chloroform/n-hexane=1:1) and further recrystallized (toluene/methanol) to obtain a white solid (2.39 g, 80%).

¹H NMR (400 MHz, CDCl₃, δ): 8.38 (s, 1H), 8.23-8.21 (m, 2H), 7.99 (d, J = 8 Hz, 1H), 7.92 (s, 1H), 7.84-7.44 (m, 15H), 7.39-7.32 (m, 3H).

MS (ASAP): 486.29 (M+H⁺). Calcd for C₃₆H₂₃NO: 485.18.

### (Synthesis Example 4) Synthesis of Compound 74

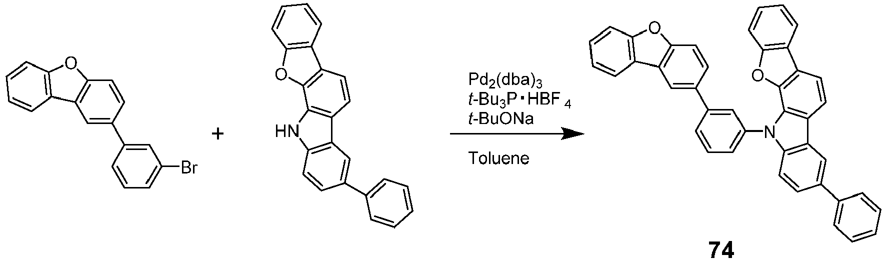

Under a nitrogen stream, a toluene solution (45 mL) of 2-(3-bromophenyl) dibenzofuran (2 g, 6.19 mmol), 3-phenyl-12H-benzofuro[2,3-a]carbazole (2.06 g, 6.19 mmol), tris(dibenzylidene acetone) dipalladium (0) (0.57 g, 0.62 mmol), tri-tert-butyl phosphonium tetrafluoroborate (0.36 g, 1.24 mmol) and sodium tert-butoxide (1.19 g, 12.4 mmol) was stirred at 130°C for 24 h. The reaction solution was cooled to room temperature, and chloroform (300 mL) was added thereto. The resultant organic layer was washed with water (300 mL) two times and dried with magnesium sulfate to remove the solvent. The resultant solid was purified with silica gel column chromatography (chloroform:hexane=1/1) and further recrystallized (toluene/methanol) to obtain Compound 74 (1.74 g, 49%).

¹H NMR (400 MHz, CDCl₃, δ): 8.43 (s, 1H), 8.32 (s, 1H), 8.11 (d, J = 8.0 Hz, 1H), 8.07 (m, 2H), 7.94-7.82 (m, 4H), 7.77-7.68 (m, 6H), 7.65 (d, J = 8.0 Hz, 1H), 7.58 (d, J = 8.0 Hz, 1H), 7.55-7.31 (m, 8H).

MS (ASAP): 576.12 (M+H⁺). Calcd for C₄₂H₂₅NO₂: 575.91.

### (Synthesis Example 5) Synthesis of Compound 82

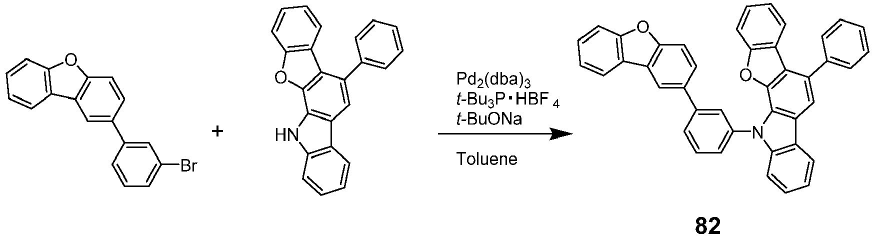

Under a nitrogen stream, a toluene solution (240 mL) of 2-(3-bromophenyl) dibenzofuran (10.6 g, 32.7 mmol), 6-phenyl-12H-benzofuro[2,3-a]carbazole (10.9 g, 32.7 mmol), tris(dibenzylidene acetone) dipalladium (0) (3.0 g, 3.2 mmol), tri-tert-butyl phosphonium tetrafluoroborate (1.9 g, 6.5 mmol), and sodium tert-butoxide (6.3 g, 65.6 mmol) was stirred at 130°C for 14 h. Toluene was added to the reaction solution and filtered with a Büchner funnel matted with celite/silica gel/celite. The solvent was removed, and then, the filtrate was washed with ethyl acetate and filtered to obtain a brown solid. The resultant solid was recrystallized with each of chlorobenzene and toluene to obtain Compound 82 (12.4 g, 65%).

¹H NMR (400 MHz, CDCl₃, δ): 8.32 (d, J = 2.0 Hz, 1H), 8.22 (d, J = 8.0 Hz, 1H), 8.07 (d, J= 1.7 Hz, 1H), 8.04 (s, 1H), 7.89-7.83 (m, 3H), 7.78-7.73 (m, 4H), 7.66 (d, J = 8.4 Hz, 2H), 7.61-7.57 (m, 3H), 7.54-7.44 (m, 5H), 7.38-7.30 (m, 3H), 7.17-7.13 (td, J = 7.4 Hz, 1.2 Hz, 1H)

MS (ASAP): 576.37 (M+H⁺). Calcd for C₄₂H₂₅NO₂: 575.19.

### (Synthesis Example 6) Synthesis of Compound 126

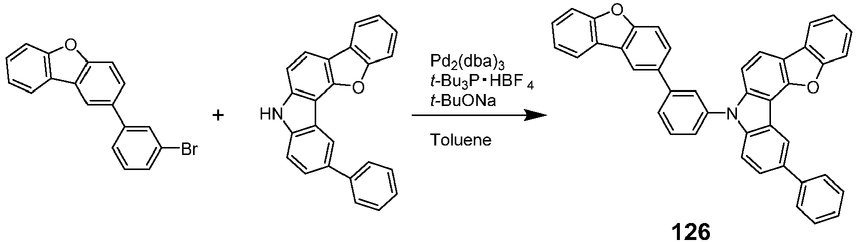

Under a nitrogen stream, a toluene solution (15 mL) of 2-(3-bromophenyl) dibenzofuran (0.97 g, 3.00 mmol), 2-phenyl-5H-benzofuro[3,2-c]carbazole (1.0 g, 3.00 mmol), tris(dibenzylidene acetone) dipalladium (0) (0.27 g, 0.30 mmol), tri-tert-butyl phosphonium tetrafluoroborate (0.17 g, 0.60 mmol), and sodium tert-butoxide (0.58 g, 6.00 mmol) was stirred at 130°C for 17 h. Chloroform was added to the reaction solution and filtered with a Buchner funnel matted with celite. The solvent was removed, and then, the filtrate was washed with chloroform and filtered to obtain a brown solid. The resultant solid was purified with column chromatography (chloroform:hexane=2:8) and further recrystallized (toluene/methanol) to obtain Compound 126 (1.09 g, 63%)
¹H NMR (400 MHz, CDCl₃, δ): 8.81 (s, 1H), 8.24 (s, 1H), 8.02-7.97 (m, 4H), 7.89-7.82 (m, 3H), 7.80-7.74 (m, 4H), 7.69-7.61 (m, 4H), 7.66-7.44 (m, 5H), 7.42-7.35 (m, 3H).

MS (ASAP): 575.21 (M⁺). Calcd for C₄₂H₂₅NO₂: 575.19.

### (Example 1)

On a glass substrate on which an anode made of an indium tin oxide (ITO) was formed with a film thickness of 50 nm, each thin film was laminated through a vacuum evaporation method at a vacuum degree of 5.0×10⁻⁵ Pa to prepare an organic electroluminescence device.

First, HAT-CN was formed with a thickness of 10 nm on ITO, and NPD was formed thereon, with a thickness of 30 nm, and further EBL1 was formed thereon, with a thickness of 10 nm. Next, a delayed fluorescence material (TADF21) and the Compound 2 were co-evaporated from different evaporation sources to form a light-emitting layer with a thickness of 40 nm. Here, the content of the delayed fluorescence material was 35% by mass, and the Compound 2 was 65% by mass. Next, SF3-TRZ was formed with a thickness of 10 nm, and then, Liq and SF3-TRZ were co-evaporated from different evaporation sources to form a layer with a thickness of 30 nm. The contents of Liq and SF3-TRZ in this layer were 30% by mass and 70% by mass, respectively. Further, Liq was formed with a thickness of 2 nm, and then, aluminum (Al) was vapor-deposited with a thickness of 100 nm to form a cathode. Then, an organic electroluminescence device was prepared as EL device 1.

Further, Comparative EL device 1 was prepared by using Comparative compound A instead of the Compound 2.

As a result of measuring the driving voltage of each of the prepared organic electroluminescence devices at a current density of 6.3 mA/cm², the driving voltage of the EL device 1 (the invention) was 0.3 V lower than the driving voltage of the Comparative EL device 1 (Comparative Example). Accordingly, it was checked that the organic electroluminescence device using the compound represented by the formula (1) as the host material of the delayed fluorescence material has excellent electrical conductivity.

### (Example 2)

An organic electroluminescence device was prepared as EL device 2 in the same procedure as in Example 1 except that TADF18 was used instead of TADF21 used in the EL device 1 of Example 1.

Similarly, an organic electroluminescence device was prepared as Comparative EL device 2 by using Comparative compound B instead of the Compound 2 used in the EL device 2.

Each of the prepared organic electroluminescence devices was driven at a current density of 12.6 mA/cm² to measure time (LT95) until luminescence intensity was 95% of that when starting the driving. As a result thereof, when LT95 of the EL device 2 was 1, LT95 of the Comparative EL device 2 was 0.6. Accordingly, it was checked that the device lifetime extends in the case of using the compound represented by the formula (1).

### (Example 3)

An organic electroluminescence device was prepared as EL device 3 in the same procedure as in Example 1 except that TADF2 was used instead of TADF21 used in the EL device 1 of Example 1.

Similarly, an organic electroluminescence device was prepared as Comparative EL device 3 by using Comparative compound B instead of the Compound 2 used in the EL device 3.

Each of the prepared organic electroluminescence devices was driven at a current density of 5.5 mA/cm² to measure time (LT95) until luminescence intensity was 95% of that when starting the driving. As a result thereof, when LT95 of the EL device 3 was 1, LT95 of the Comparative EL device 3 was 0.8. Accordingly, it was checked that the device lifetime extends in the case of using the compound represented by the formula (1).

### (Example 4)

An organic electroluminescence device was prepared as EL device 4 in the same procedure as in Example 1 except that the Compound 2 was used instead of EBL1 used in the Comparative EL device 1 (Comparative Example) of Example 1, and as in Example 1, a driving voltage at a current density of 6.3 mA/cm² was measured. The driving voltage of the EL device 4 (the invention) was 0.23 V lower than the driving voltage of the Comparative EL device 1 (Comparative Example). Accordingly, it was checked that the compound represented by the formula (1) is useful as an electron barrier material.

The driving voltage of an organic electroluminescence device prepared in the same procedure as in Example 1 except that Reference compound a was used instead of EBL1 used in the Comparative EL device 1 (Comparative Example) of Example 1 was also lower than that of the Comparative EL device 1, but was not lower than that of the EL device 4, and there was an obvious difference.

### (Example 5)

On a glass substrate on which an anode made of an indium tin oxide (ITO) was formed with a film thickness of 50 nm, each thin film was laminated through a vacuum evaporation method at a vacuum degree of 5.0×10⁻⁵ Pa to prepare an organic electroluminescence device.

First, HAT-CN was formed with a thickness of 10 nm on ITO, and NPD was formed thereon, with a thickness of 30 nm, and further Tris-PCz was formed thereon, with a thickness of 10 nm. Next, a delayed fluorescence material (TADF72) and the Compound 74 were co-evaporated from different evaporation sources to form a light-emitting layer with a thickness of 40 nm. Here, the content of the delayed fluorescence material was 35% by mass, and the content of the Compound 74 was 65% by mass. Next, SF3-TRZ was formed with a thickness of 10 nm, and then, Liq and SF3-TRZ were co-evaporated from different evaporation sources to form a layer with a thickness of 30 nm. The contents of Liq and SF3-TRZ in this layer were 30% by mass and 70% by mass, respectively. Further, Liq was formed with a thickness of 2 nm, and then, aluminum (Al) was vapor-deposited with a thickness of 100 nm to form a cathode. Then, an organic electroluminescence device was prepared as EL device 5.

EL device 6 was prepared by using Compound 82 instead of the Compound 74, and EL device 7 was prepared by using Compound 126 instead of the Compound 74.

In each of the prepared organic electroluminescence device, the driving voltage at a current density of 6.3 mA/cm², the external quantum efficiency (EQE), and the device lifetime (LT95) at a current density of 12.6 mA/cm² were measured. The results are noted in the following table, which are relative values when the EL device 6 was set as a reference. According to the results of the table, it was checked that all of the organic electroluminescence devices using the compound represented by the formula (1) as the host material have excellent electrical conductivity, high luminous efficiency, and long lifetime.

**Table 1**

| | Host material | Driving voltage (relative value) | EQE (relative magnification) | LT95 (relative magnification) |
|---|---|---|---|---|
| EL device 5 | Compound 74 | -0.09 V | 0.94 magnification | 1.47 magnification |
| EL device 6 | Compound 82 | 0 | 1 | 1 |
| EL device 7 | Compound 126 | -0.09 V | 1.07 magnification | 1.03 magnification |

### (Example 6)

On a glass substrate on which an anode made of an indium tin oxide (ITO) was formed with a film thickness of 50 nm, each thin film was laminated through a vacuum evaporation method at a vacuum degree of 5.0×10⁻⁵ Pa to prepare an organic electroluminescence device.

First, HAT-CN was formed with a thickness of 10 nm on ITO, and NPD was formed thereon, with a thickness of 30 nm, and further the Compound 2 was formed thereon, with a thickness of 10 nm. Next, the delayed fluorescence material (TADF21) and the Comparative compound A were co-evaporated from different evaporation sources to form a light-emitting layer with a thickness of 40 nm. Here, the content of the delayed fluorescence material was 35% by mass, and the content of the Comparative compound A was 65% by mass. Next, SF3-TRZ was formed with a thickness of 10 nm, and then, Liq and SF3-TRZ were co-evaporated from different evaporation sources to form a layer with a thickness of 30 nm. The contents of Liq and SF3-TRZ in this layer were 30% by mass and 70% by mass, respectively. Further, Liq was formed with a thickness of 2 nm, and then, aluminum (Al) was vapor-deposited with a thickness of 100 nm to form a cathode. Then, an organic electroluminescence device was prepared as EL device 8.

Further, EL device 9 was prepared by using Compound 30 instead of the Compound 2.

Further, Comparative EL device 5 was prepared by using the Comparative compound A instead of the Compound 2.

In each of the prepared organic electroluminescence devices, the driving voltage at a current density of 6.3 mA/cm² and the external quantum efficiency (EQE) were measured. The results are noted in the following table, which are relative values when the EL device 5 was set as a reference. According to the results of the table, it was checked that all of the organic electroluminescence devices using the compound represented by the formula (1) as the electron barrier material have excellent electrical conductivity and high luminous efficiency.

**Table 2**

| | Electron barrier material | Driving voltage (relative value) | EQE (relative magnification) |
|---|---|---|---|
| EL device 8 | Compound 2 | -0.81 V | 1.11 magnification |
| EL device 9 | Compound 30 | -0.81 V | 1.07 magnification |
| Comparative EL device 5 | Comparative compound A | 0 | 1 |

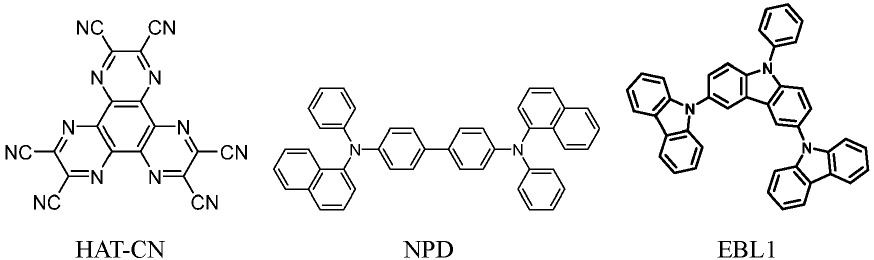

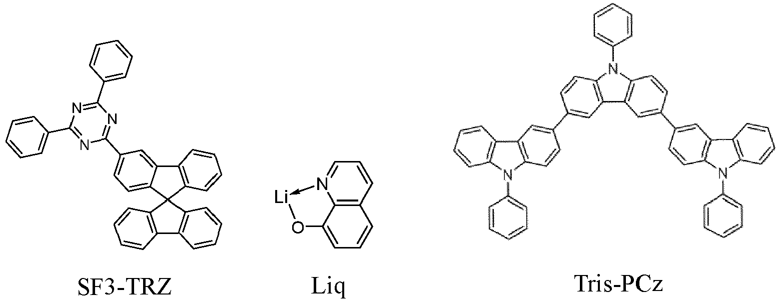

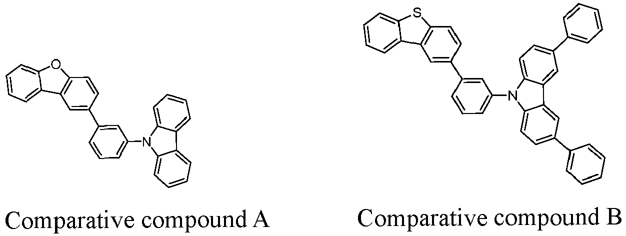

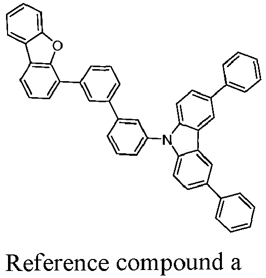

### Reference Signs List

1: Substrate
2: Anode
3: Hole injection layer
4: Hole transport layer
5: Light-emitting layer
6: Electron transport layer
7: Cathode

## Claims

1. A compound represented by the following formula (1), 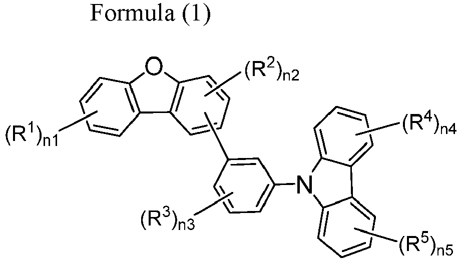 wherein each of R¹ to R³ independently represents a deuterium atom, or an alkyl group that may be deuterated, each of R⁴ and R⁵ independently represents a deuterium atom, an alkyl group that may be deuterated, or a phenyl group that may be substituted with an alkyl group that may be deuterated or a deuterium atom; here, one or two among R⁴ and R⁵ are a phenyl group that may be substituted with an alkyl group that may be deuterated or a deuterium atom; R¹ to R⁵ are not bonded to other R¹ to R⁵ to form a ring structure, and adjacent R¹'s, adjacent R²'s, and adjacent R³'s are not bonded to each other to form a ring structure; adjacent R⁴'s may be bonded to each other to form a benzofuro skeleton or a benzothieno skeleton, and adjacent R⁵'s may be bonded to each other to form a benzofuro skeleton or a benzothieno skeleton; and each of n1, n3, n4, and n5 independently represents any integer of 0 to 4, n2 represents any integer of 0 to 3, and a sum of n4 and n5 is 1 to 8.

2. The compound according to claim 1, wherein adj acent R⁴'s and adjacent R⁵'s are not bonded to each other to form a ring structure.

3. The compound according to claim 1, wherein adjacent R⁵'s are bonded to each other to form a benzofuro skeleton or a benzothieno skeleton.

4. The compound according to any one of claims 1 to 3, wherein each of R⁴ and R⁵ is independently a deuterium atom or a phenyl group, and the phenyl group may be substituted with a deuterium atom, or an alkyl group that may be deuterated.

5. The compound according to any one of claims 1 to 4, wherein each of only one R⁴ and one R⁵ is independently a phenyl group that may be substituted with an alkyl group that may be deuterated or a deuterium atom.

6. The compound according to any one of claims 1 to 4, wherein only one R⁴ is a phenyl group that may be substituted with an alkyl group that may be deuterated or a deuterium atom.

7. The compound according to any one of claims 1 to 6, wherein R¹ to R³ are a deuterium atom.

8. The compound according to any one of claims 1 to 7, wherein at least one of R⁴'s is a deuterium atom, and at least one of R⁵'s is a deuterium atom.

9. The compound according to any one of claims 1 to 8, wherein nl to n3 are 0.

10. The compound according to any one of claims 1 to 9, wherein dibenzofuran in the formula (1) is bonded to a phenylene group in the formula (1) at a 2-position.

11. The compound according to any one of claims 1 to 10, wherein the compound is represented by the following formula (2), 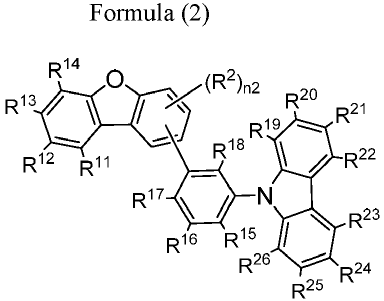 wherein R² represents an alkyl group that may be deuterated, and n2 represents an integer of 0 to 3; each of R¹¹ to R¹⁸ independently represents a hydrogen atom, a deuterium atom, or an alkyl group that may be deuterated; each of R¹⁹ to R²⁶ independently represents a hydrogen atom, a deuterium atom, an alkyl group that may be deuterated, or a phenyl group that may be substituted with an alkyl group that may be deuterated or a deuterium atom; here, one or two among R¹⁹ to R²⁶ are a phenyl group that may be substituted with an alkyl group that may be deuterated or a deuterium atom; and R¹⁹ and R²⁰, R²⁰ and R²¹, R²¹ and R²², R²³ and R²⁴, R²⁴ and R²⁵, and R²⁵ and R²⁶ may be bonded to each other to form a benzofuro skeleton or a benzothieno skeleton.

12. The compound according to claim 11, wherein none of adjacent R²'s, R¹¹ and R¹², R¹² and R¹³ , R¹³ and R¹⁴, R¹⁵ and R¹⁶, R¹⁶ and R¹⁷, R¹⁹ and R²⁰, R²⁰ and R²¹, R²¹ and R²², R²³ and R²⁴, R²⁴ and R²⁵, and R²⁵ and R²⁶ are bonded to each other to form a ring structure.

13. The compound according to claim 11 or 12, wherein at least one of R²¹ and R²⁴ is a phenyl group that may be substituted with an alkyl group that may be deuterated or a deuterium atom.

14. A host material comprising the compound according to any one of claims 1 to 13.

15. The host material according to claim 14 for use together with a delayed fluorescence material.

16. An electron barrier material comprising the compound according to any one of claims 1 to 13.

17. A composition comprising the compound according to any one of claims 1 to 13 and a dopant of a delayed fluorescence material.

18. The composition according to claim 17, which is in the shape of a film.

19. The composition according to claim 17 or 18, wherein the delayed fluorescence material is a compound having a cyanobenzene structure in which the number of cyano groups substituted with the benzene ring is 1.

20. The composition according to claim 17 or 18, wherein the delayed fluorescence material is a compound having a dicyanobenzene structure in which the number of cyano groups substituted with the benzene ring is 2.

21. The composition according to any one of claims 17 to 20, further comprising a fluorescent compound having lowest excited singlet energy lower than that of the host material and the delayed fluorescence material.

22. An organic light-emitting device comprising a layer containing the composition according to any one of claims 17 to 21.

23. The organic light-emitting device according to claim 22, wherein the layer consists of atoms selected from the group consisting of a carbon atom, a hydrogen atom, a nitrogen atom, an oxygen atom, a sulfur atom, a boron atom, and a halogen atom.

24. The organic light-emitting device according to claim 23, wherein the layer consists of atoms selected from the group consisting of a carbon atom, a hydrogen atom, a nitrogen atom, an oxygen atom, and a sulfur atom.

25. The organic light-emitting device according to any one of claims 22 to 24, which is an organic electroluminescence device.

26. The organic light-emitting device according to any one of claims 22 to 25, wherein the composition does not contain the fluorescent compound, and the largest component of light emitted from the device is light emitted from the delayed fluorescence material.

27. The organic light-emitting device according to any one of claims 22 to 25, wherein the composition contains the fluorescent compound, and the largest component of light emitted from the device is light emitted from the fluorescent compound.
